# EUROPEAN PATENT APPLICATION

(11) **EP 4 397 685 A1**
(43) Date of publication of application: **10.07.2024**
(21) Application number: 21955620.6
(22) Date of filing: 14.09.2021
(51) Int. Cl.: C07K 16/28, C07K 19/00, C12N 15/10, C12N 15/13, A61K 39/395, A61K 47/68, A61P 35/00, A61P 35/02

(54) **ANTI-CD3 HUMANIZED ANTIBODY**

(30) Priority: 03.09.2021 CN 202111028884; 03.09.2021 CN 202111028883
(71) Applicant: Novoprotein Scientififc Inc., Suzhou, Jiangsu 215200 (CN); Novoprotein Scientific (Shanghai) Inc., Shanghai 201203 (CN)
(72) Inventor: ZHU, Huaxing, Suzhou, Jiangsu 215200 (CN); SONG, Zuowei, Suzhou, Jiangsu 215200 (CN); LI, Debin, Suzhou, Jiangsu 215200 (CN); YU, Futao, Suzhou, Jiangsu 215200 (CN); QIN, Zhaofeng, Shanghai 201203 (CN); FANG, Jixuan, Shanghai 201203 (CN); WANG, Mi, Shanghai 201203 (CN)
(74) Representative: Lavoix
(86) International application number: PCT/CN2021/118264
(87) International publication number: WO 2023/029089

(57) **Abstract**

Provided is a novel anti-CD3 humanized antibody. The anti-CD3 humanized antibody can specifically bind to human and monkey CD3 proteins, and said antibody has good biological activity.

## Description

### Technical field

The present invention relates to the field of biomedicine, specifically to an anti-CD3 humanized antibody.

### Background

The activation of T cells is crucial for stimulating immune responses. T cells exhibit immune specificity and guide most cellular immune responses. Although T cells do not secrete antibodies, they are necessary for B lymphocytes to secrete antibodies.

CD3 is a homodimeric or heterodimeric antigen expressed on T cells that is associated with T cell receptor complexes (TCRs) and is essential for T cell activation. Functional CD3 is a dimer formed by two of four different chains < ε,ζ,δ and γ) . The arrangement of CD3 dimers includes γ/ε,δ/ε and ζ/ζ. It has been confirmed that anti-CD3 antibodies aggregate CD3 on T cells, causing T cell activation in a manner similar to the involvement of peptide loaded MHC molecules in TCR. Therefore, anti-CD3 antibodies have therapeutic purposes involving T cell activation. In addition, bispecific antibodies that can bind to CD3 and target antigens have been proposed for therapeutic purposes involving targeting T cell immune responses to tissues and cells expressing target antigens.

The existing bispecific antibodies that bind to CD3, which are currently in the clinical trial stage of cancer treatment, are limited due to their short half-life and/or uncertain efficacy. Although many attempts have been made to obtain anti-CD3 antibodies with particularly advantageous properties, so far , the success of these attempts has been limited.

Therefore, there is still an urgent need to develop a novel anti-CD3 humanized antibody in this field.

### Summary of the invention

The purpose of the invention is to provide a novel anti-CD3 humanized antibody .

In the first aspect of the invention, it provides an anti-CD3 humanized antibody, wherein the antibody comprises a heavy chain variable region and a light chain variable region, wherein the heavy chain variable region and the light chain variable region have six complementary determining regions CDRs selected from the following group:
(A5) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH5-CDR3: HGNFGNTYISYWAY, | SEQ ID NO.29 |
| HuVL5 CDR1: TGAVTSGNY, | SEQ ID NO.30 |
| HuVL-CDR2: GTK, | SEQ ID NO.5 |
| HuVL5 CDR3: VLWYSKRW, | SEQ ID NO.31; |

(A1) three complementary determining regions VH-CDRs in the heavy chain variable region and three complementary determining regions VL-CDRs in the light chain variable region:

| | |
|---|---|
| HuVH-CDR1: GFTFNKYA, | SEQ ID NO. 1 |
| HuVH-CDR2: IRSKYNNYAT, | SEQ ID NO. 2 |
| HuVH4-CDR3: HGNFGNSYISYWEY, | SEQ ID NO. 3 |
| HuVL-CDR1: TGAVTSGNY, | SEQ ID NO. 4 |
| HuVL-CDR2: GTK, | SEQ ID NO. 5 |
| HuVL4-CDR3: VLWNSNRW, | SEQ ID NO. 6; |

(A2) three complementary determining regions VH-CDRs in the heavy chain variable region and three complementary determining regions VL-CDRs in the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH3-CDR3: HGNFGNSYISYWRY, | SEQ ID NO.10 |
| HuVL-CDR1: TGAVTSGNY, | SEQ ID NO.4 |
| HuVL-CDR2: GTK, | SEQ ID NO.5 |
| HuVL3-CDR3: VLWYSGRW, | SEQ ID NO.11; |

(A3) three complementary determining regions VH-CDRs in the heavy chain variable region and three complementary determining regions VL-CDRs in the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH2-CDR3: HGNFGNSYISYWQY, | SEQ ID NO.15 |
| HuVL-CDR1: TGAVTSGNY, | SEQ ID NO.4 |
| HuVL-CDR2: GTK, | SEQ ID NO.5 |
| HuVL2-CDR3: VLWRSNRW, | SEQ ID NO.16;; |

or
(A4) three complementary determining regions VH-CDRs in the heavy chain variable region and three complementary determining regions VL-CDRs in the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH1-CDR3: HGNFGNTYISYWAY, | SEQ ID NO.20 |
| HuVL-CDR1: TGAVTSGNY, | SEQ ID NO.4 |
| HuVL-CDR2: GTK, | SEQ ID NO.5 |
| HuVL1-CDR3: VLWYSKRW, | SEQ ID NO.21. |

In another preferred embodiment, any one of the above amino acid sequences also includes a derivative sequence that is optionally with at least one amino acid added, deleted, modified, and/or substituted, and is capable of retaining the binding affinity of CD3.

In another preferred embodiment, the number of added, deleted, modified, and/or substituted amino acids is 1-5 (e.g. 1-3, preferably 1-2, preferably 1).

In another preferred embodiment, the derivative sequence with at least one amino acid added, deleted, modified, and/or substituted, which can retain the binding affinity of CD3, is an amino acid sequence having a homology or sequence identity of at least 96%. In another preferred embodiment, the antibody comprises a heavy chain and a light chain, wherein the heavy chain of the antibody comprises three heavy chain CDRs and a heavy chain framework region for connecting the heavy chain CDR, and the light chain of the antibody comprises three light chain CDRs and a light chain framework region for connecting the light chain CDR.

In another preferred embodiment, the sequence of the heavy chain variable region is as shown in SEQ ID NO. 7.

In another preferred embodiment, the heavy chain of the antibody further comprises heavy chain constant region.

In another preferred embodiment, the heavy chain constant region is of human, mouse, or rabbit, preferably of human.

In another preferred embodiment, the sequence of the light chain variable region is as shown in SEQ ID NO. 8.

In another preferred embodiment, the light chain of the antibody further comprises a light chain constant region.

In another preferred embodiment, the light chain constant region is of human, mouse, or rabbit, preferably of human.

In another preferred embodiment, the Kd of the antibody binding to human CD3 ε peptide is ≤ 250nM, preferably ≤ 100nM; preferably; ≤ 15nM; preferably, ≤ 10nM; more preferably, ≤ 5nM.

In another preferred embodiment, the antibody specifically binds to CD3.

In another preferred embodiment, the CD3 derived from human or cynomolgus monkey.

In another preferred embodiment, the CD3 is CD3 ε, CD3 ζ, CD3 δ or CD3 γ; preferably, the CD3 is CD3 ε or CD3 γ.

In another preferred embodiment, the antibody specifically binds to human CD3 ε or cynomolgus monkey CD3 ε; or the antibody specifically binds to human CD3 γ or cynomolgus monkey CD3 γ.

In another preferred embodiment, the antibody is a double chain antibody or a single chain antibody (scFv).

In another preferred embodiment, the antibody is a single chain antibody, wherein the single chain antibody comprises a heavy chain variable region and a light chain variable region, wherein the heavy chain variable region and light chain variable region have six complementary determining regions CDRs selected from the following group:
(A5) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH5-CDR3: HGNFGNTYISYWAY, | SEQ ID NO.29 |
| HuVL5 CDR1: TGAVTSGNY, | SEQ ID NO.30 |
| HuVL-CDR2: GTK, | SEQ ID NO.5 |
| HuVL5 CDR3: VLWYSKRW, | SEQ ID NO.31; |

(A1) three complementary determining regions VH-CDRs of the heavy chain variable regions and three complementary determining regions VL-CDRs of the light chain variable region:

| | |
|---|---|
| HuVH-CDR1: GFTFNKYA, | SEQ ID NO. 1 |
| HuVH-CDR2: IRSKYNNYAT, | SEQ ID NO. 2 |
| HuVH4-CDR3: HGNFGNSYISYWEY, | SEQ ID NO. 3 |
| HuVL-CDR1: TGAVTSGNY, | SEQ ID NO. 4 |
| HuVL-CDR2: GTK, | SEQ ID NO. 5 |
| HuVL4-CDR3: VLWNSNRW, | SEQ ID NO. 6; |

(A2) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH3-CDR3: HGNFGNSYISYWRY, | SEQ ID NO.10 |
| HuVL-CDR1: TGAVTSGNY, | SEQ ID NO.4 |
| HuVL-CDR2: GTK, | SEQ ID NO.5 |
| HuVL3-CDR3: VLWYSGRW, | SEQ ID NO.11; |

(A3) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH2-CDR3: HGNFGNSYISYWQY, | SEQ ID NO.15 |
| HuVL-CDR1: TGAVTSGNY, | SEQ ID NO.4 |
| HuVL-CDR2: GTK, | SEQ ID NO.5 |
| HuVL2-CDR3: VLWRSNRW, | SEQ ID NO.16; |

or
(A4) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH1-CDR3: HGNFGNTYISYWAY, | SEQ ID NO.20 |
| HuVL-CDR1: TGAVTSGNY, | SEQ ID NO.4 |
| HuVL-CDR2: GTK, | SEQ ID NO.5 |
| HuVL1-CDR3: VLWYSKRW, | SEQ ID NO.21. |

In another preferred embodiment, the single chain antibody sequentially comprises a light chain variable region, a linker, and a heavy chain variable region, or sequentially comprises a heavy chain variable region, a linker, and a light chain variable region.

In another preferred embodiment, the linker is (G4S) n, where n is an integer from 1 to 5, and the preferred linker is (G4S) 3.
In another preferred embodiment, the heavy chain variable region of the antibody has the amino acid sequence shown in SEQ ID NO. 32; and the light chain variable region of the antibody has the amino acid sequence shown in SEQ ID NO. 33; or
The heavy chain variable region of the antibody has the amino acid sequence shown in SEQ ID NO. 7; and the light chain variable region of the antibody has the amino acid sequence shown in SEQ ID NO. 8; or
The heavy chain variable region of the antibody has the amino acid sequence shown in SEQ ID NO. 12; and the light chain variable region of the antibody contains the amino acid sequence shown in SEQ ID NO. 13; or
The heavy chain variable region of the antibody has the amino acid sequence shown in SEQ ID NO. 17; and the light chain variable region of the antibody has the amino acid sequence shown in SEQ ID NO. 18; or
The heavy chain variable region of the antibody has the amino acid sequence shown in SEQ ID NO. 22; and the light chain variable region of the antibody contains the amino acid sequence shown in SEQ ID NO. 23.

In another preferred embodiment, the sequence of the single chain antibody is selected from the following groups:
SEQ ID NO. 34, SEQ ID NO. 9, SEQ ID NO. 22, SEQ ID NO. 19, or SEQ ID NO. 24.

In another preferred embodiment, the amino acid sequence of the heavy chain variable region has a sequence homology or sequence identity of at least 80%, 85%, 90%, 91%, 92%, 93%, 94%, 95%, 96%, 97%, 98%, or 99% with the amino acid sequence shown in SEQ IDs NO.32, 7, 12, 17, and 22.

In another preferred embodiment, the amino acid sequence of the light chain variable region has a sequence homology or sequence identity of at least 80%, 85%, 90%, 91%, 92%, 93%, 94%, 95%, 96%, 97%, 98%, or 99% with the amino acid sequence shown in SEQ ID NO.33, 8, 13, 18, 23.

In another preferred embodiment, the antibody is a monoclonal antibody.

In another preferred embodiment, the antibodies comprises single specific, bispecific, or trispecific antibodies.

In another preferred embodiment, the bispecific antibody comprises:
(1) anti-CD3 antibody according to the first aspect of the invention;
(2) antibodies that binding to other targets.

In another preferred embodiment, the other targets are selected from the following groups: BCMA, EGFR, CD38, CD123, CD19, CD20, CD22, B7-H3, GPC3, HER2, PMSA, CD28, 4-1BB, OX40, CD40, CD27, CD47, CTLA4, PD1, PDL1.

In another preferred embodiment, the bispecific antibody comprises:
the first antigen binding domain (D1); and
the second antigen binding domain (D2);
wherein, D1 specifically binds to the target molecule CD3 protein;
D2 specifically binds to the target molecule CD19 protein;
wherein, the D1 is an antibody or antigen-binding fragment that specifically binds to CD3 protein;
the D2 is an antibody or antigen-binding fragment that specifically binds to CD19 protein;
wherein, the D1 comprises a heavy chain variable region and a light chain variable region, wherein the heavy chain variable region and the light chain variable region have six complementary determining regions CDRs selected from the following group:
(A5) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH5-CDR3: HGNFGNTYISYWAY, | SEQ ID NO.29 |
| HuVL5 CDR1: TGAVTSGNY, | SEQ ID NO.30 |
| HuVL-CDR2: GTK, | SEQ ID NO.5 |
| HuVL5 CDR3: VLWYSKRW, | SEQ ID NO.31; |

(A1) three complementary determining regions VH-CDR of the heavy chain variable region and three complementary determining regions VL-CDRs of the light chain variable region:

| | |
|---|---|
| HuVH-CDR1: GFTFNKYA, | SEQ ID NO. 1 |
| HuVH-CDR2: IRSKYNNYAT, | SEQ ID NO. 2 |
| HuVH4-CDR3: HGNFGNSYISYWEY, | SEQ ID NO. 3 |
| HuVL-CDR1: TGAVTSGNY, | SEQ ID NO. 4 |
| HuVL-CDR2: GTK, | SEQ ID NO. 5 |
| HuVL4-CDR3: VLWNSNRW, | SEQ ID NO. 6; |

(A2) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH3-CDR3: HGNFGNSYISYWRY, | SEQ ID NO.10 |
| HuVL-CDR1: TGAVTSGNY, | SEQ ID NO.4 |
| HuVL-CDR2: GTK, | SEQ ID NO.5 |
| HuVL3-CDR3: VLWYSGRW, | SEQ ID NO.11; |

(A3) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH2-CDR3: HGNFGNSYISYWQY, | SEQ ID NO.15 |
| HuVL-CDR1: TGAVTSGNY, | SEQ ID NO.4 |
| HuVL-CDR2: GTK, | SEQ ID NO.5 |
| HuVL2-CDR3: VLWRSNRW, | SEQ ID NO.16; |

or
(A4) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH1-CDR3: HGNFGNTYISYWAY, | SEQ ID NO.20 |
| HuVL-CDR1: TGAVTSGNY, | SEQ ID NO.4 |
| HuVL-CDR2: GTK, | SEQ ID NO.5 |
| HuVL1-CDR3: VLWYSKRW, | SEQ ID NO.21; |

wherein, the structure of the antigen binding fragment is selected from the following group: (i) Fab fragment; (ii) F (ab ') ₂ fragment; (iii) Fd fragments; (iv) Fv fragments; (v) ScFv molecules; or (vi) dAb fragments.

In another preferred embodiment, the D1 and/or D2 are single chain antibodies (scFv).

In another preferred embodiment, the D1 and D2 are connected through a linker, preferably a flexible linker.

In another preferred embodiment, the linker is (GGGGS) n, where n is an integer from 1 to 5, and the preferred linking peptide is GGGGS.

In another preferred embodiment, the D1 is an anti-CD3 single chain antibody, and D2 is an anti-CD19 single chain antibody.
In another preferred embodiment, the anti-CD3 single chain antibody comprises a heavy chain variable region and a light chain variable region, wherein the heavy chain variable region comprises the amino acid sequence shown in SEQ ID NO. 32; and the light chain variable region of the antibody comprises the amino acid sequence shown in SEQ ID NO. 33;
The variable region of the heavy chain has the amino acid sequence shown in SEQ ID NO. 7; and the variable region of the light chain has the amino acid sequence shown in SEQ ID NO. 8; or
The variable region of the heavy chain has the amino acid sequence shown in SEQ ID NO. 12; and the variable region of the light chain has the amino acid sequence shown in SEQ ID NO. 13; or
The variable region of the heavy chain has the amino acid sequence shown in SEQ ID NO. 17; and the variable region of the light chain has the amino acid sequence shown in SEQ ID NO. 18; or
The variable region of the heavy chain has the amino acid sequence shown in SEQ ID NO. 22; and the variable region of the light chain has the amino acid sequence shown in SEQ ID NO. 23.

In another preferred embodiment, the sequence of the bispecific antibody is as shown in SEQ ID NO. 37.

In the second aspect of the invention, a recombinant protein is provided, wherein the recombinant protein comprises:
(i) the antibody or its bispecific antibody of the first aspect of the invention; and
(ii) optional tag sequences for assisted expression and/or purification.

In another preferred embodiment, the tag sequence comprises a 6His tag.

In another preferred embodiment, the recombinant protein (or peptide) comprises a fusion protein.

In another preferred embodiment, the recombinant protein is a monomer, dimer, or polymer.

In another preferred embodiment, the recombinant protein is a single specific, double specific, or triple specific recombinant protein.

In another preferred embodiment, the recombinant protein further comprises (i) additional fusion elements (or fusion peptide fragments) fused with the element.

In another preferred embodiment, the recombinant protein comprises:
(i) an antibody selected from the following group, wherein,
   the heavy chain variable region of the antibody has the amino acid sequence shown in SEQ ID NO. 32; and the light chain variable region of the antibody has the amino acid sequence shown in SEQ ID NO. 33; or
   the heavy chain variable region of the antibody has the amino acid sequence shown in SEQ ID NO. 7; and the light chain variable region of the antibody has the amino acid sequence shown in SEQ ID NO. 8; or
   the heavy chain variable region of the antibody has the amino acid sequence shown in SEQ ID NO. 12; and the light chain variable region of the antibody has the amino acid sequence shown in SEQ ID NO. 13; or
   the heavy chain variable region of the antibody has the amino acid sequence shown in SEQ ID NO. 17; and the light chain variable region of the antibody has the amino acid sequence shown in SEQ ID NO. 18; or
   the heavy chain variable region of the antibody has the amino acid sequence shown in SEQ ID NO. 22; and the light chain variable region of the antibody has the amino acid sequence shown in SEQ ID NO. 23;
   and
(ii) optional tag sequences for assisted expression and/or purification.

In a third aspect of the invention, a CAR construct is provided, wherein the scFv segment of the antigen binding region of the CAR construct is specifically bind to the CD3 binding region, and the scFv segment has a heavy chain variable region and a light chain variable region, wherein the heavy chain variable region and light chain variable region have six complementary determining regions CDRs selected from the following group:
(A5) three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH5-CDR3: HGNFGNTYISYWAY, | SEQ ID NO.29 |
| HuVL5 CDR1: TGAVTSGNY, | SEQ ID NO.30 |
| HuVL-CDR2: GTK, | SEQ ID NO.5 |
| HuVL5 CDR3: VLWYSKRW, | SEQ ID NO.31; |

(A1) The three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO. 1 |
| HuVH-CDR2: IRSKYNNYAT, | SEQ ID NO. 2 |
| HuVH4-CDR3: HGNFGNSYISYWEY, | SEQ ID NO. 3 |
| HuVL-CDR1: TGAVTSGNY, | SEQ ID NO. 4 |
| HuVL-CDR2: GTK, | SEQ ID NO. 5 |
| HuVL4-CDR3: VLWNSNRW, | SEQ ID NO. 6; |

(A2) The three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH3-CDR3: HGNFGNSYISYWRY, | SEQ ID NO.10 |
| HuVL-CDR1: TGAVTSGNY, | SEQ ID NO.4 |
| HuVL-CDR2: GTK, | SEQ ID NO.5 |
| HuVL3-CDR3: VLWYSGRW, | SEQ ID NO.11; |

(A3) The three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH2-CDR3: HGNFGNSYISYWQY, | SEQ ID NO.15 |
| HuVL-CDR1: TGAVTSGNY, | SEQ ID NO.4 |
| HuVL-CDR2: GTK, | SEQ ID NO.5 |
| HuVL2-CDR3: VLWRSNRW, | SEQ ID NO.16; |

or
(A4) The three complementary determining regions VH-CDRs of the heavy chain variable region and three complementary determining regions VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH1-CDR3: HGNFGNTYISYWAY, | SEQ ID NO.20 |
| HuVL-CDR1: TGAVTSGNY, | SEQ ID NO.4 |
| HuVL-CDR2: GTK, | SEQ ID NO.5 |
| HuVL1-CDR3: VLWYSKRW, | SEQ ID NO.21. |

In the fourth aspect of the invention, a recombinant immune cell is provided, which expresses an exogenous CAR construct of the third aspect of the invention.

In another preferred embodiment, the immune cells are selected from the following group: NK cells, T cells.

In another preferred embodiment, the immune cells are derived from human or non-human mammals (such as mice).

In a fifth aspect of the invention, an antibody drug conjugate is provided, which comprising:
(a) an antibody portion, wherein the antibody portion is selected from the following group: the antibody or its bispecific antibody of the first aspect of the invention, the recombinant protein of the second aspect of the invention, or combination thereof; and
(b) a coupling moiety coupled to the antibody moiety, and the coupling moiety is selected from the group consisting of a detectable label, a drug, a toxin, a cytokine, a radionuclide, an enzyme, or a combination thereof.

In another preferred embodiment, the antibody moiety is coupled to the coupling moiety via a chemical bond or linker.

In a sixth aspect of the invention, the use of an active ingredient is provided, wherein the active ingredient is selected from the following group: the antibody or its bispecific antibody of the first aspect of the invention, the recombinant protein of the second aspect of the invention, the CAR construct of the third aspect of the invention, the immune cell of the fourth aspect of the invention, the antibody drug conjugate of the fifth aspect of the invention, or combination thereof, wherein the active ingredient is used for:
(a) preparing a test reagent or test kit;
(b) preparing drugs or preparations for the prevention and/or treatment of CD3 related diseases; and/or
(c) preparing drugs or preparations for the prevention and/or treatment of CD3 related cancers or tumors.

In another preferred embodiment, the cancer or tumor is selected from the following groups: lung cancer, melanoma, colon cancer, pancreatic cancer, bladder cancer, breast cancer, ovarian cancer, prostate cancer, testicular cancer, esophageal cancer, stomach cancer, liver cancer, lymphoma, myeloma, leukemia.

In a seventh aspect of the invention, it provides a pharmaceutical composition comprises:
(i) active ingredients selected from the following groups: the antibodies or bispecific antibodies of the first aspect of the invention, the recombinant proteins of the second aspect of the invention, the CAR constructs of the third aspect of the invention, the immune cells of the fourth aspect of the invention, the antibody drug conjugates of the fifth aspect of the invention, or combinations thereof; and
(ii) a pharmaceutically acceptable carrier.

In another preferred embodiment, the pharmaceutical composition is a liquid preparation.

In another preferred embodiment, the pharmaceutical composition is an injection.

In another preferred embodiment, the drug composition is used to prepare drugs for treating cancer, the cancer is selected from the following groups: lung cancer, melanoma, colon cancer, pancreatic cancer, bladder cancer, breast cancer, ovarian cancer, prostate cancer, testicular cancer, esophageal cancer, stomach cancer, liver cancer, lymphoma, myeloma, leukemia.

In the eighth aspect of the invention, a polynucleotide is provided, which encodes a polypeptide selected from the following group:
(1) the antibody or its bispecific antibody of the first aspect of the invention; or
(2) the recombinant protein of the second aspect of the invention;
(3) the CAR construct of the third aspect of the invention.

In the ninth aspect of the invention, a vector is provided, wherein the carrier comprises the polynucleotide of the eighth aspect of the invention.

In another preferred embodiment, the vector comprises: a bacterial plasmid, a bacteriophage, a yeast plasmid, a plant cell virus, a mammalian cell virus such as an adenovirus, a retrovirus, or other vectors.

In the tenth aspect of the invention, a genetically engineered host cell is provided, wherein the host cell comprises the vector of the ninth aspect of the invention or the genome integrated with a polynucleotide of the eighth aspect of the invention.

In another preferred embodiment, the host cell is mammalian cell or prokaryotic cell.

In another preferred embodiment, the mammalian cell is Chinese hamster ovary (CHO) cells.

In another preferred embodiment, the prokaryotic cell is *Escherichia coli.*

In the eleventh aspect of the invention, an *in vitro* method non-diagnostic detection of CD3 protein in samples is provided, wherein the method comprises the steps:
(1) contacting the sample with the antibody of the first aspect of the present invention *in vitro*;
(2) detecting the formation of an antigen-antibody complex, wherein the formation of a complex indicates the presence of CD3 protein in the sample.

In a twelfth aspect of the invention, it provides a detection plate comprising: a substrate (support plate) and a test strip, wherein the test strip comprises the antibody of the first aspect of the invention or the antibody drug conjugate of the fifth aspect of the invention.

In the thirteenth aspect of the invention, it provides a kit, which comprises:
(1) the first container containing the antibody of the first aspect of the present invention; and/or
(2) the second container containing the secondary antibody against the antibody of the first aspect of the present invention;
alternatively, the kit comprises the detection plate of the twelfth aspect of the invention.

In the fourteenth aspect of the invention, it provides a method for preparing a recombinant polypeptide, which comprises the steps:
(a) culturing the host cell of the tenth aspect of the invention under conditions suitable for expression;
(b) isolating a recombinant polypeptide from the culture, wherein the recombinant polypeptide is the antibody of the first aspect of the present invention or the recombinant protein of the second aspect of the present invention.

In a fifteenth aspect of the invention, it provides a method for treating diseases related to CD3 molecules, which comprises the steps: administering the antibody of the first aspect of the invention, the recombinant protein of the second aspect of the invention, or the pharmaceutical composition of the seventh aspect of the invention to a subject in need of inhibition or treatment.

In another preferred embodiment, the CD3 molecules associated diseases comprises tumors or antagonistic organ transplant immune rejection reactions.

In another preferred embodiment, the subject is mammals (including humans).

It should be understood that within the scope of the present invention, the various technical features of the present invention above and the various technical features specifically described hereinafter (as in the embodiments) may be combined with each other to constitute a new or preferred technical solution. Due to space limitations, it is not repeated here.

### Description of Drawings

Figure 1 shows the affinity diagram of CD3 antibodies and recombinant human CD3 determined by ELISA.
Figure 2 shows the affinity diagram of CD3 antibodies and recombinant monkey CD3 determined by ELISA.
Figure 3 shows the affinity diagram of CD3 full-length antibodies and human CD3E protein determined by Fortebio, wherein A, B, C, D, and E are the affinity diagram of CD3 full-length antibodies CQ53, CQ52, CQ51, CQ50, CQ54 with human CD3E protein, respectively.
Figure 4 shows the affinity diagram of CD3 full-length antibodies with monkey CD3E protein determined by ELISA, wherein A, B, C, D, and E are the affinity diagrams of CD3 full-length antibodies CQ53, CQ52, CQ51, CQ50, CQ54 with monkey CD3E protein, respectively.
Figure 5 shows the affinity diagrams of CD3 full-length antibodies and Jurkat cells, wherein A, B, C, D, and E are the affinity diagrams of CD3 full-length antibodies CQ53, CQ52, CQ51, CQ50, CQ54 with Jurkat cells, respectively.
Figure 6 shows the competitive ELISA detection diagrams of CD3 full-length antibodies and OKT3, wherein A, B, C, D, and E are the competitive ELISA detection diagrams of CD3 full-length antibodies CQ53, CQ52, CQ51, CQ50, CQ54 withOKT3, respectively.
Figure 7 shows the change in CD69 expression induced by CD3 antibody, with NC as the negative control.
Figure 8 shows the diagram of CD3 antibody induced PBMC activation and secretion of IFN γ, wherein NC is a negative control.
Figure 9 shows the affinity diagrams of bispecific antibodies with human CD3E (A) and CD19 (B) proteins determined by fortebio.
Figure 10 shows the affinity diagram of bispecific antibodies (SC30A and BLMOA) and monkey CD3E protein determined by ELISA.
Figure 11 shows the change in CD69 expression induced by CD3 antibody, wherein NC is the negative control.
Figure 12 shows the killing efficiency of dual specific antibodies (SC30A and BLMOA) on tumor cells detected by flow cytometry.
Figure 13 shows the residual image of Raji-GFP displayed by fluorescence scanning.
Figure 14 shows the efficiency of direct killing of PBMC tumor cells mediated by bispecific antibodies.
Figure 15 shows the diagram of proliferation experiment of PBMC.
Figure 16 shows the dependence of the reporter gene detection SC30A signal on target cells, with RLU as the relative light unit.
Figure 17 shows the activity map of the reporter gene detection SC30A, with RLU as the relative light unit.

### Embodiment

After extensive and in-depth research, the inventor unexpectedly obtained highly biologically active CD3 antibodies. Experiments have shown that the CD3 antibody of the invention can specifically bind to human and monkey CD3. The CD3 antibody of the invention can further construct bispecific antibodies with antibodies or their binding fragments that bind to the target antigen CD19, exhibiting higher tumor killing activity, thereby achieving therapeutic use of T cell immune response targeting tissues and cells expressing target antigen cells. In addition, the activation of the bispecific antibody of the invention depends on the target cell and has safety. On this basis, the invention has been completed.

### The terms

As used in herein, the terms "administration" and "treatment" refer to the application of exogenous drugs, therapeutic agents, diagnostic agents, or combinations to animals, humans, subjects, cells, tissues, organs, or biological fluids. "Administration" and "treatment" may refer to therapy, drug metabolism dynamics, diagnostics, studies, and experimental methods. The treatment of the cell includes the contact of the reagent with the cell, and the contact of the reagent with the fluid, the contact of the fluid with the cell. "Administration" and "treatment" also mean *in vitro* and *ex vivo* treatment by reagents, diagnostics, binding compositions, or by another cell. "Treatment ", when applied to a human, animal, or research subject, refers to therapeutic, prophylactic or prophylactic measures, studies and diagnostics; and it comprises contactinganti-CD3 antibody with a human or animal, subject, cell, tissue, physiological compartment, or physiological fluid.

As used herein, the term "treating" refers to the administration of an internal or external therapeutic agent comprising any one of the CD3 antibodies and a composition thereof of the present invention to a patient having one or more disease symptoms for which the therapeutic agent is known to have a therapeutic effect. Generally, the amount of a therapeutic agent that effectively relieves one or more disease symptoms (therapeutically effective amount) is administrated to a patient.

As used herein, the terms "optional" or "optionally" mean that the subsequently described event or circumstance may occur but not necessarily. For example, "optionally contains 1-3 antibody heavy chain variable regions" refers to the specific sequence of the antibody heavy chain variable region can have, but not necessarily, may be 1, 2 or 3.

### Antibody

As used herein, the term " antibody " refers to an immunoglobulin, which is a tetrapeptide chain structure formed by connecting two identical heavy chains and two identical light chains through an inter-chain disulfide bond. The amino acid composition and arrangement sequence of the immunoglobulin heavy chain constant region are different, so the antigenicity of the immunoglobulin heavy chain constant region is also different. Accordingly, immunoglobulins may be classified into five classes, or different types of immunoglobulins, ie, IgM, IgD, IgG, IgA and IgE. The heavy chain constant regions corresponding to different types of immunoglobulins are called α, δ, ε, γ, and µ, respectively. IgG represents the most important class in immunoglobulin, which in turn can be divided into four subclasses: IgG1, IgG2, IgG3 and IgG4 due to differences in chemical and biological functions. The light chain is divided into κ or λ chains through different constant regions. The subunit structure and three-dimensional configuration of different classes of immunoglobulins are well known to those in the art.

The sequence of about 110 amino acids near the N terminal of the heavy chain and light chain of antibody changed greatly, which was the variable region (V region). The remaining amino acid sequence near the C terminal is relatively stable, which is the constant region (C region). The variable regions include three highly variable regions (HVRs) and four FR regions (FRs) with relatively conserved sequences. The 4 FRs amino acid sequences are relatively conservative and do not directly participate in the binding reaction. The three highly variable regions determine the specificity of the antibody, and are also referred to as complementarity determining regions (CDRs)). Each light chain variable region (LCVR) and the heavy chain variable region (HCVR) are composed of three CDR regions and four FR regions (frame regions), and sequentially arranged from the amino terminal to the carboxyl terminal are FR1, CDR1, FR2, CDR2, FR3, CDR3, and FR4. The three CDR regions of the light chain, ie the light chain highly variable region (LCDR), refer to LCDR1, LCDR2 and LCDR3; and the three CDR regions of the heavy chain, i.e. the heavy chain highly variable region (HCDR), refer to HCDR1, HCDR2 and HCDR3. The CDR amino acid residues of the LCVR and HCVR regions of the antibody or antigen-binding fragment of the invention conform in number and position to known Kabat numbering rules (LCDR1-3, HCDR2-3), or conform to the kabat and chothia numbering rules (HCDR1). Four FR regions of the variable regions of naturally occurring heavy and light chains are generally in a-sheet configuration, joined by the three CDRs forming a linking loop, and in some cases, may form a partical -sheet structure. The CDRs in each chain get close through the FR regions and together with the CDRs of the other chain form the antigen-binding site of the antibody. It can be determined which amino acids constitute the FR or CDR region by comparing the amino acid sequences of antibodies of the same type. Constant regions are not directly involved in the binding of antibodies to an antigen, however, they exhibit different effector functions, such as participating in the antibody-dependent cytotoxicity of antibodies.

As used herein, "antigen-binding domain" and "single-chain antibody fragment" both refer to a Fab fragment, a Fab' fragment, a F (ab')₂ fragment, or a single Fv fragment with antigen-binding activity. Fv antibody contains antibody heavy chain variable region and light chain variable region, but no constant region, and has the smallest antibody fragment of all antigen binding sites. In general, Fv antibody further contains a polypeptide linker between the VH and VL domains and is capable of forming the structure required for antigen binding. Non-restrictive examples of antigen binding fragments include: (i) Fab fragments; (ii) F (ab ')₂ fragment; (iii) Fd fragments; (iv) Fv fragments; (v) ScFv molecules; (vi) dAb fragments; (vii)the smallest recognition unit composed of amino acid residues that mimic the high variability region of antibodies (e.g., independent complementary determining regions (CDRs) such as CDR3 peptides) or constrained FR3-CDR3-FR4 peptides. As used in herein, the description "antigen binding fragment" also includes other engineered molecules, such as domain specific antibodies, single domain antibodies, domain deletion antibodies, chimeric antibodies, CDR transplantation antibodies, bispecific antibodies, trisomy antibodies, tetrasomic antibodies, micro antibodies, nanobodies (such as monovalent and divalent nanobodies), small module immunodrugs (SMIP), and shark variable IgNAR domains.

As used herein, the term "antigenic determinant" refers to a three-dimensional spatial site on an antigen that is not contiguous and is recognized by an antibody or antigen-binding fragment of the present invention.

The present invention includes not only intact antibodies, but also fragments of immunologically active antibodies or fusion proteins formed by antibodies with other sequences. Thus, the present invention also includes fragments, derivatives and analogs of the antibody.

In the present invention, the antibodies include murine, chimeric, humanized or full-human antibodies prepared with techniques well known to those skilled in the art. Recombinant antibodies, such as chimeric and humanized monoclonal antibodies, including human and non-human parts, can be prepared using DNA recombinant techniques well known in the art.

As used herein, the term "monoclonal antibody" refers to an antibody secreted from a single cell-derived clone. The monoclonal antibody is highly specific for a single antigenic epitope. The cells may be eukaryotic, prokaryotic or cloned cell strains of phage.

As used herein, the term "chimeric antibody" is an antibody molecule that is formed by splicing a V-region gene of a murine antibody and a C-region gene of a human antibody into a chimeric gene, then inserting a vector, and transfecting a host cell. The high specificity and affinity of the parent mouse antibody are retained, and the human Fc segment can effectively mediate the biological effect function.

As used herein, the term "humanized antibody" is a variable region modification form of a murine antibody of the present invention, having a CDR region derived (or substantially derived from) a non-human antibody (preferably a mouse monoclonal antibody), and an FR region and a constant region substantially derived from a human antibody sequence; ie. grafting the CDR region sequence of the mouse antibody onto different types of human-based antibody framework sequences. Because CDR sequences are responsible for most antibody-antigen interactions, recombinant antibodies that mimic specific naturally occurring antibody properties can be expressed by constructing an expression vector.

In the present invention, the antibody may be monospecific, bispecific, trispecific, or more multiple specificities.

In the present invention, the antibody of the present invention further includes a conservative variant thereof, which means a polypeptide formed by replacing at most 10, preferably at most 8, more preferably at most 5, and most preferably at most 3 amino acids with amnio acid of similar properties as compared with the amino acid sequence of the antibody of the present invention. These conservative variant polypeptides are better produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lvs |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-CD3 antibody

As used in herein, the term "CD3" generally refers to natural or recombinant human CD3, as well as non-human homologs of human CD3. CD3 is a homodimeric or heterodimeric antigen expressed on T cells that is associated with T cell receptor complexes (TCRs) and is essential for T cell activation. Functional CD3 is a dimer formed by two of four different chains ( ε,ζ,δ and γ) . The arrangement of CD3 dimers includes γ/ε,δ/ε and ζ/ζ. Therefore, unless it is explicitly stated that it comes from non-human species, such as "mouse CD3", "monkey CD3", etc., the term "CD3" refers to human CD3.

As used in herein, "CD3E" refers to CD3 ε extracellular domain. The present invention provides a highly specific single chain antibody (scFv) targeting CD3, comprising a heavy chain variable region (VH), a light chain variable region (VL), and a linker for connecting.

The present invention also provides a highly specific antibody against CD3, comprising a heavy chain and a light chain, wherein the heavy chain contains a heavy chain variable region (VH) amino acid sequence, and the light chain contains a light chain variable region (VL) amino acid sequence.

Preferably, the heavy chain variable region and the light chain variable region have six complementary determining regions CDR selected from the following group:
(A5) The three complementary determining regions VH-CDRs of the heavy chain variable region and VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH5-CDR3: HGNFGNTYISYWAY, | SEQ ID NO.29 |
| HuVL5 CDR1: TGAVTSGNY, | SEQ ID NO.30 |
| HuVL-CDR2: GTK, | SEQ ID NO.5 |
| HuVL5 CDR3: VLWYSKRW, | SEQ ID NO.31; |

(A1) The three complementary determining regions VH-CDRs of the heavy chain variable region and VL-CDRs of the light chain variable region:

| | |
|---|---|
| HuVH-CDR1: GFTFNKYA, | SEQ ID NO. 1 |
| HuVH-CDR2: IRSKYNNYAT, | SEQ ID NO. 2 |
| HuVH4-CDR3: HGNFGNSYISYWEY, | SEQ ID NO. 3 |
| HuVL-CDR1: TGAVTSGNY, | SEQ ID NO. 4 |
| HuVL-CDR2: GTK, | SEQ ID NO. 5 |
| HuVL4-CDR3: VLWNSNRW, | SEQ ID NO. 6; |

(A2) The three complementary determining regions VH-CDRs of the heavy chain variable region and VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH3-CDR3: HGNFGNSYISYWRY, | SEQ ID NO.10 |
| HuVL-CDR1: TGAVTSGNY, | SEQ ID NO.4 |
| HuVL-CDR2: GTK, | SEQ ID NO.5 |
| HuVL3-CDR3: VLWYSGRW, | SEQ ID NO.11; |

(A3) The three complementary determining regions VH-CDRs of the heavy chain variable region and VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH2-CDR3: HGNFGNSYISYWQY, | SEQ ID NO.15 |
| HuVL-CDR1: TGAVTSGNY, | SEQ ID NO.4 |
| HuVL-CDR2: GTK, | SEQ ID NO.5 |
| HuVL2-CDR3: VLWRSNRW, | SEQ ID NO.16; |

or
(A4) The three complementary determining regions VH-CDRs of the heavy chain variable region and VL-CDRs of the light chain variable region:

| | |
|---|---|
| HuVH-CDR1: GFTFNKYA, | SEQ ID NO.1 |
| HuVH-CDR2: IRSKYNNYAT, | SEQ ID NO.2 |
| HuVH1-CDR3: HGNFGNTYISYWAY, | SEQ ID NO.20 |
| HuVL-CDR1: TGAVTSGNY, | SEQ ID NO.4 |
| HuVL-CDR2: GTK, | SEQ ID NO.5 |
| HuVL1-CDR3: VLWYSKRW, | SEQ ID NO.21. |

Preferably, the heavy chain variable region of the antibody has the amino acid sequence shown in SEQ ID NO. 32; and the light chain variable region of the antibody contains the amino acid sequence shown in SEQ ID NO. 33; or
the sequence of the heavy chain variable region includes an amino acid sequence as shown in SEQ ID NO. 7; and the sequence of the light chain variable region includes an amino acid sequence as shown in SEQ ID NO. 8; or
the variable region of the heavy chain has the amino acid sequence shown in SEQ ID NO. 12; and the variable region of the light chain contains the amino acid sequence shown in SEQ ID NO. 13; or
the variable region of the heavy chain has the amino acid sequence shown in SEQ ID NO. 17; and the variable region of the light chain has the amino acid sequence shown in SEQ ID NO. 18; or
the variable region of the heavy chain has the amino acid sequence shown in SEQ ID NO. 22; and the variable region of the light chain contains the amino acid sequence shown in SEQ ID NO. 23.
wherein, any one of the amino acid sequences mentioned above also includes a derivative sequence with CD3 binding affinity that has been added, deleted, modified, and/or replaced with at least one (such as 1-5, 1-3, preferably 1-2, preferably 1) amino acid.

In another preferred embodiment, the sequence with at least one amino acid added, deleted, modified and/or substituted in any of the above amino acid sequences is preferably an amino acid sequence having a homology of at least 80%, more preferably at least 85%, more preferably at least 90%, most preferably at least 95% to the above amino acid sequence.

The antibody of the present invention may be a double-chain or single-chain antibody, and may be selected from animal-derived antibodies, chimeric antibodies and humanized antibodies, more preferably be selected from humanized antibodies and human-animal chimeric antibodies, more preferably a fully humanized antibody.

As used in herein, the term "scFv" refers to a single chain antibody fragment (scFv), which is typically formed by linking the heavy chain variable region and light chain variable region by 15-25 amino acids linker.

As used herein, the term "linker" refers to one or more amino acid residues inserted into the immunoglobulin domain to provide sufficient mobility for the domain of light and heavy chains to fold into exchange for dual variable domain immunoglobulins. In the invention, the preferred linker refers to a linker that connects the VH and VL of a single chain antibody (scFv), or is used to connect scFv to a heavy chain of another antibody.

Examples of suitable linkers include monoglycine (Gly), or serine (Ser) residues, and the identification and sequence of amino acid residues in the linker may vary with the type of secondary structural element to be implemented in the linker. For example, (G4S) n, wherein n is an integer from 1 to 5.

The antibody derivatives of the present invention may be single chain antibodies, and/or antibody fragments, such as: Fab, Fab', (Fab')₂ or other known antibody derivatives in the art, etc., as well as any one or several of IgA, IgD, IgE, IgG and IgM antibodies or other subtypes.

Wherein, the animal is preferably a mammal, such as a rat.

The antibody of the present invention may be a mouse antibody, a chimeric antibody, a humanized antibody, a CDR grafted and/or modified antibody targeting human CD3.

In a preferred embodiment of the invention, the above

| |
|---|
| SEQ ID NO. 1, SEQ ID NO. 2, and SEQ ID NO. 3; |
| SEQ ID NO. 1, SEQ ID NO. 2, and SEQ ID NO. 10; |
| SEQ ID NO. 1, SEQ ID NO. 2, and SEQ ID NO. 15; |
| SEQ ID NO. 1, SEQ ID NO. 2, and SEQ ID NO. 20; |
| SEQ ID NO. 1, SEQ ID NO. 2, and SEQ ID NO. 29 |

or
or a sequence with at least one amino acid added, deleted, modified and/or substituted thereof and with CD3 binding affinity is located in the CDR region of the heavy chain variable region (VH).

In a preferred embodiment of the invention, the above

| |
|---|
| SEQ ID NO. 4, SEQ ID NO. 5, and SEQ ID NO. 6; |
| SEQ ID NO. 4, SEQ ID NO. 5, and SEQ ID NO. 11; |
| SEQ ID NO. 4, SEQ ID NO. 5, and SEQ ID NO. 16; |
| SEQ ID NO. 4, SEQ ID NO. 5, and SEQ ID NO. 21; |
| SEQ ID NO. 30, SEQ ID NO. 5, and SEQ ID NO. 31 |

or
or a sequence with at least one amino acid added, deleted, modified and/or substituted thereof and with CD3 binding affinity is located in the CDR region of the light chain variable region (VL).

In the above content of the present invention, the number of added, deleted, modified and/or substituted amino acids is preferably not more than 40% of the total number of amino acids in the original amino acid sequence, more preferably not more than 35%, more preferably 1-33%, more preferably 5-30%, more preferably 10-25%, more preferably 15-20%.

In the present invention, the number of added, deleted, modified and/or substituted amino acids is usually 1, 2, 3, 4, or 5, preferably 1-3, more preferably 1-2, optimally 1.

As used in herein, the term "bispecific antibody (or bispecific antibody)" refers to an antibody molecule that can specifically bind two antigens (targets) or two epitopes simultaneously. According to symmetry, bispecific antibodies can be divided into structurally symmetric and asymmetric molecules. According to the number of binding sites, bispecific antibodies can be divided into divalent, trivalent, tetravalent, and multivalent molecules.

The antibodies of the invention can be single specific, bispecific, trispecific, or multispecific. For example, the antibodies of the invention can form bispecific antibodies together with antibodies or active fragments that bind to other targets. The antibodies that bind to other targets can be antibodies targeting CD19, CD47, CD73, CD47, CTLA4, PD-1, PD-L1, CD28 or their active fragments.

In a specific embodiment of the present invention, the anti-CD3 antibody and the antibody binding to CD19 form a bispecific antibody, wherein the bispecific antibody targeting CD3 and CD19 is named SC30A, and the sequence is shown in SEQ ID NO. 37.

### Preparation of Antibodies

Any method suitable for producing monoclonal antibodies can be used to produce the CD3 antibody of the present invention. For example, an animal may be immunized with a linked or naturally occurring CD3 protein or a fragment thereof. Suitable immune inoculation methods may be used, including adjuvants, immunostimulatory agents, repeated enhanced immune inoculation, and one or more approaches may be used.

Any suitable form of CD3 can be used as an immunogen (antigen) for producing a non-human antibody specific to CD3 and screening the biological activity of the antibody. The stimulating immunogen can be full-length mature human CD3, including natural homologous dimers or peptides containing single/multiple epitopes. The immunogen may be used alone or in combination with one or more immunogenic enhancers known in the art. The immunogen may be purified from a natural source, or produced in genetically modified cells. The DNA encoding the immunogen may be a genomic or non-genome (eg, cDNA) on the source. DNA encoding an immunogen may be expressed using a suitable genetic vector including, but not limited to, an adenovirus vector, a baculovirus vector, a plasmid, and a non-viral vector.

An exemplary method of preparing the anti humanCD3 antibody of the present invention is described in Example 1.

Humanized antibodies may be selected from any kind of immunoglobulins, including IgM, IgD, IgG, IgA, and IgE. In the invention, the antibody is an IgG antibody, using the IgG1 subtype. By using the biological assays described in the following embodiments to screen antibodies, it is easy to optimize the necessary constant domain sequence to generate the desired biological activity.

Similarly, any type of light chain can be used in the compounds and methods described in this article. Specifically, κ,λ Chains or their variants may be used in the compounds and methods of the invention.

An exemplary method for humanizing the anti human CD3 antibody of the invention is described in Example 2.

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence. Then, the DNA sequence can be introduced into various existing DNA molecules (or vectors) and cells known in this field.

The present invention also relates to vectors containing appropriate DNA sequences and appropriate promoters or control sequences as described above. These vectors can be used to transform appropriate host cells to enable them to express proteins.

Host cells may be prokaryotic cells, such as bacterial cells; or lower eukaryotic cells, such as yeast cells; or higher eukaryotic cells, such as mammalian cells. Preferred animal cells include (but are not limited to) CHO-S, CHO-K1, and HEK-293 cells.

The step of transforming host cells with recombinant DNA described in the invention can be carried out using techniques well-known in the art. The obtained transformant can be cultured by conventional methods to express the polypeptide encoded by the gene of the present invention. According to the host cells used, culture is performed under suitable conditions with conventional culture media.

In general, under conditions suitable for expression of the antibody according to the present invention, the host cell obtained is cultured. Then, the antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an in vitro binding assay (such as radioimmunoassay (RIA) or enzyme-linked immunosorbent assay (ELISA)).

### Antibody-Drug Conjugate (ADC)

The present invention also provides antibody drug conjugates (ADCs) based on the antibodies of the invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH3COO⁻, Cl⁻ or NOs ; and polymethylene dimethyl sulfide thiosulfonate. The linker may include, for example, a maleimide linked to an antibody via thiosuccimide.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. In the invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

### Application

The present invention provides use of the antibody according to the present invention, for example, for preparation of a diagnostic agent, or for preparation of a medicament for preventing and/or treating a CD3 associated disease. The CD3 related diseases including inflammatory diseases, autoimmune diseases, etc., including but not limited to psoriasis, psoriatic arthritis, ankylosing spondylitis, multiple sclerosis, inflammatory bowel diseases (such as Crohn's disease, ulcerative colitis, etc.), osteoarthritis, rheumatoid arthritis (RA), rheumatoid arthritis or osteoporosis, inflammatory fibrosis (such as scleroderma, pulmonary fibrosis and sclerosis), asthma (including allergic asthma), allergies, and cancer.

### Pharmaceutical composition

The present invention also provides a composition. In the preferred examples, the composition is a pharmaceutical composition comprising the antibody, or an active fragment, a fusion protein or an ADC thereof, or a corresponding CAR-T cell, and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to) intratumoral, intraperitoneal, intravenous, or topical administration.

The antibody of the present invention can also be used for cell therapy by expressing the nucleotide sequence in the cell. For example, the antibody is used for chimeric antigen receptor T cell immunotherapy (CAR-T) and the like.

The pharmaceutical composition according to the present invention can be directly used for binding to a CD3 protein molecule, and thus can be used for preventing and treating diseases related CD3. In addition, other therapeutic agents may be used at the same time.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include (but are not limited to): saline, buffers, glucose, water, glycerol, ethanol, and a combination thereof. The drug formulation should match the administration method. The pharmaceutical composition of the present invention can be prepared in the form of injection, for example, prepared by a conventional method using physiological saline or an aqueous solution containing glucose and other adjuvants. The pharmaceutical composition such as an injection and solution should be manufactured under sterile conditions. The dosage of active ingredient is therapeutically effective amount, for example from about 1 microgram per kilogram body weight to about 5 milligrams per kilogram body weight per day. In addition, the polypeptide according to the present invention may also be used in combination with an additional therapeutic agent.

When a pharmaceutical composition is used, a safe and effective amount of pharmaceutical composition is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, the particular dose should also depend on various factors, such as the route of administration, patient healthy status, which are well within the skills of an experienced physician.

### Detection application and kit

The antibodies of the invention can be used in detection applications, for example, for detecting a sample to provide diagnostic information.

In the present invention, the samples (samples) used include cells, tissue samples and biopsy specimens. The term "biopsy" used in the present invention shall include all kinds of biopsy known to those skilled in the art. Therefore, the biopsy used in the present invention can include tissue samples prepared, for example, through endoscopic methods or organ puncture or needle biopsy.

Samples for use in the present invention include fixed or preserved cell or tissue samples.

The present invention also provides a kit containing the antibody (or a fragment thereof) of the invention. InThe present invention also provides a kit containing the antibody (or a fragment thereof) of the present invention. In a preferred embodiment of the present invention, the kit further includes a container, instructions for use, buffer, and the like. In a preferred example, the antibody of the present invention can be immobilized on a detection plate.

### The main advantages of the present invention include:

(1) The CD3 antibody of the invention specifically binds to CD3 and has high biological activity;
(2) The CD3 antibody of the invention can be used to construct bispecific antibodies with other antibodies.

The invention is further illustrated below in conjunction with specific embodiments. It is to be understood that these examples are for illustrative purposes only and are not intended to limit the scope of the invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Percentages and parts are by weight unless otherwise stated.

### Example 1 Production of humanized CD3 monoclonal antibody

Balb/C mice were immunized through recombinant human CD3E protein (C00E, Novoprotein). After immunization, B cells were taken to construct a phage display library. Candidate antibodies were obtained by screening, and a series of humanized antibodies were obtained through structural simulation and rational design. The obtained humanized antibody heavy chain variable regions were huVH1, huVH2, huVH3, and huVH4, while the obtained humanized antibody light chain variable regions were huVL1, huVL2, huVL3, and huVL4. Connected the VH and VL of the humanized sequence through a (G4S) 3 linker and a 6HIS tag was added at the C-terminus to construct a single chain antibody form (scFv). Expressed it in *Escherichia coli* and purified it on a nickel column to obtain the single chain antibody protein. The obtained humanized antibody heavy chain variable region (VH), light chain variable region (VL) sequences, and CDR are as follows:
wherein, VH-CDR1 and VH-CDR2 were both the same, with the following sequence:
VH-CDR1: GFTFNKYA (SEQ ID NO.1)
VH-CDR2: IRSKYNNYAT (SEQ ID NO. 2);
The VH-CDR3 sequences have differences, as follows:
   HuVH5-CDR3: HGNLQNSYISYWAY (SEQ ID NO. 29)
   HuVH4-CDR3: HGNFGNTYISYWAY (SEQ ID NO. 3)
   HuVH3-CDR3: HGNFGNSYISYWQY (SEQ ID NO. 10)
   HuVH2-CDR3: HGNFGNSYISYWRY (SEQ ID NO. 15)
   HuVH1-CDR3: HGNFGNSYISYWEY (SEQ ID NO. 20).
Wherein, the VL-CDR1 sequences of huVL1-VL4 were the same, as follows:
   VL-CDR1: TGAVTSGNY (SEQ ID NO.4);
The VL5 CDR1 of huVL5 had changed, with the following sequence:
   HuVL5 CDR1: TGPVTGGNY (SEQ ID NO. 30)
The VL-CDR2 sequences of huVL1-VL5 were the same, as follows:
   VL-CDR2: GTK (SEQ ID NO. 5);
The VL-CDR3 sequences have differences, as follows:
   HuVL5-CDR3: VLWESNRW (SEQ ID NO. 31)
   HuVL4 CDR3: VLWYSKRW (SEQ ID NO.6)
   HuVL3-CDR3: VLWRSNRW (SEQ ID NO.11)
   HuVL2-CDR3: VLWYSGRW (SEQ ID NO.16)
   HuVL1-CDR3: VLWNSNRW (SEQ ID NO.21).
The heavy chain variable region huVH5 of humanized CD3 antibody (SEQ ID NO. 32) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNLQ
NSYISYWAYWGQGTLVTVSS
The heavy chain variable region huVH4 of humanized CD3 antibody (SEQ ID NO. 7) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNFG
NSYISYWEYWGQGTLVTVSS
The heavy chain variable region huVH3 of humanized CD3 antibody (SEQ ID NO.12) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNFG
NSYISYWRYWGQGTLVTVSS
The heavy chain variable region huVH2 of humanized CD3 antibody (SEQ ID NO.17) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNFG
NSYISYWQYWGQGTLVTVSS
The heavy chain variable region huVH1 of humanized CD3 antibody (SEQ ID NO.22) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNFG
NTYISYWAYWGQGTLVTVSS
The light chain variable region huVL5 of humanized CD3 antibody (SEQ ID NO.33) 
QTVVTQEPSLTVSPGGTVTLTCGSSTGPVTGGNYPNWVQQKPGQAPRGLIGG
TKFLAPGTPARFSGSLLGGKAALTLSGVQPEDEAEYYCVLWESNRWVFGGGTKLT
VL
The light chain variable region huVL4 of humanized CD3 antibody (SEQ ID NO.8) 
QTVVTQEPSLTVSPGGTVTLTCGSSTGAVTSGNYPNWVQQKPGQAPRGLIGG
TKFLAPGTPARFSGSLLGDKAALTLSGVQPEDEAEYYCVLWNSNRWVFGGGTKLT
VL
The light chain variable region huVL3 of humanized CD3 antibody (SEQ ID NO.13) 
QTVVTQEPSLTVSPGGTVTLTCGSSTGAVTSGNYPNWVQQKPGQAPRGLIGG
TKFLAPGTPARFSGSLLGGKAALTLSGVQPEDEAEYYCVLWYSGRWVFGGGTKLT
VL
The light chain variable region huVL2 of humanized CD3 antibody (SEQ ID NO.18) 
QTVVTQEPSLTVSPGGTVTLTCGSSTGAVTSGNYPNWVQQKPGQAPRGLIGG
TKFLAPGTPARFSGSLLGGKAALTLSGVQPEDEAEYYCVLWRSNRWVFGGGTKLT
VL
The light chain variable region huVL1 of humanized CD3 antibody (SEQ ID NO.23) 
QTVVTQEPSLTVSPGGTVTLTCGSSTGAVTSGNYPNWVQQKPGQAPRGLIGG
TKFLAPGTPARFSGSLLGGKAALTLSGVQPEDEAEYYCVLWYSKRWVFGGGTKLT
VL
Humanized CD3 single chain antibody huVH5VL5 (SEQ ID NO. 34) 
QTVVTQEPSLTVSPGGTVTLTCGSSTGPVTGGNYPNWVQQKPGQAPRGLIGG
TKFLAPGTPARFSGSLLGGKAALTLSGVQPEDEAEYYCVLWESNRWVFGGGTKLT
VLTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
Humanized CD3 single chain antibody huVH4VL4 (SEQ ID NO. 9) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNFG
NSYISYWEYWGQGTLVTVSSGGGGSGGGGSGGGGSQTVVTQEPSLTVSPGGTVTL
TCGSSTGAVTSGNYPNWVQQKPGQAPRGLIGGTKFLAPGTPARFSGSLLGDKAALT
LSGVQPEDEAEYYCVLWNSNRWVFGGGTKLTVL
Humanized CD3 single chain antibody huVH3VL3 (SEQ ID NO. 14) EVQLVESGGGLVQPGGSLKLSCAASGFTFNKYAMNWVRQAPGKGLEWVARI 
RSKYNNYATYYADSVKDRFTISRDDSKNTAYLQMNNLKTEDTAVYYCVRHGNFG
NSYISYWRYWGQGTLVTVSSGGGGSGGGGSGGGGSQTVVTQEPSLTVSPGGTVTL
TCGSSTGAVTSGNYPNWVQQKPGQAPRGLIGGTKFLAPGTPARFSGSLLGGKAALT
LSGVQPEDEAEYYCVLWYSGRWVFGGGTKLTVL
Humanized CD3 single chain antibody huVH2VL2 (SEQ ID NO. 19) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNFG
NSYISYWQYWGQGTLVTVSSGGGGSGGGGSGGGGSQTVVTQEPSLTVSPGGTVTL
TCGSSTGAVTSGNYPNWVQQKPGQAPRGLIGGTKFLAPGTPARFSGSLLGGKAALT
LSGVQPEDEAEYYCVLWRSNRWVFGGGTKLTVL
Humanized CD3 single chain antibody huVHIVL1 (SEQ ID NO. 24)

EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNFG
NTYISYWAYWGQGTLVTVSSGGGGSGGGGSGGGGSQTVVTQEPSLTVSPGGTVTL
TCGSSTGAVTSGNYPNWVQQKPGQAPRGLIGGTKFLAPGTPARFSGSLLGGKAALT
LSGVQPEDEAEYYCVLWYSKRWVFGGGTKLTVL

### Example 2 Affinity detection of CD3 antibody

The affinity detection of CD3 antibodies in the form of single chain antibodies with recombinant CD3E proteins in humans and monkeys were described.

### 2.1 Affinity with human CD3 protein

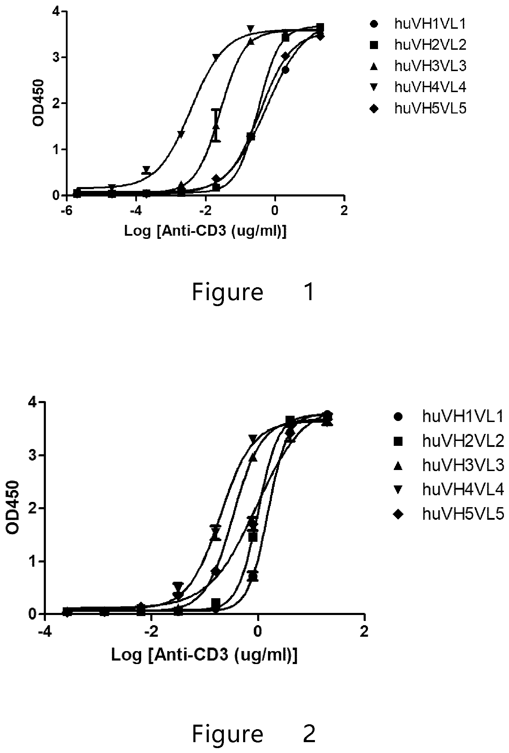

ELISA was used to determine the affinity between CD3 antibody and recombinant hCD3 protein (CP19, Novoprotein). The recombinant hCD3 was coated on a plate and the CD3 antibody was subjected to a 10 fold gradient (from 20µg/ml) dilution, as shown in Figure 1.

The calculated EC50 results were shown in Table 1.

**Table 1. EC50 of human CD3 protein affinity for CD3 antibodies in the form of single chain antibodies**

| | HuVH1VL1 | HuVH2VL2 | HuVH3VL3 | HuVH4VL4 | HuVH5VL5 |
|---|---|---|---|---|---|
| EC50(µg/ml) | 0.5609 | 0.3203 | 0.02591 | 0.003782 | 0.3642 |

### 2.2 Affinity with Monkey CD3 Protein

ELISA was used to determine the affinity between CD3 antibody and recombinant monkey CD3 protein (CW07, Novoprotein). The recombinant monkey CD3 was coated on a plate and the CD3 antibody was subjected to a 10 fold gradient (from 20µg/ml) dilution, as shown in Figure 2.

The calculated EC50 results were shown in Table 2.

**Table 2. EC50 affinity between CD3 antibodies in the form of single chain antibodies and monkey CD3 protein**

| | HuVH1VL1 | HuVH2VL2 | HuVH3VL3 | HuVH4VL4 | HuVH5VL5 |
|---|---|---|---|---|---|
| EC50 ( µg/ml) | 1.527 | 0.9898 | 0.3418 | 0.197 | 0.9381 |

### Example 3 Full-length antibody and Construction of huVH5VL5 bispecific antibodies.

A CD3 full-length antibody was constructed by fusing the heavy chain variable region with the heavy chain constant region hIgG1, and fusing the light chain variable region with the light chain constant region kappa chain. The obtained CD3 full-length antibodies were CQ50 (including huVH1 and huVL1), CQ51 (including huVH2 and huVL2), CQ52 (including huVH3 and huVL3), CQ53 (including huVH4 and huVL4), and CQ54 (including huVH5 and huVL5), respectively. The variable region of the marketed drug OKT3 was chosed and was fused with hIgG1 as the control antibody. The relevant sequence as follows:
**Heavy chain of CD3 full-length antibody CQ54 (SEQ ID NO. 35)** 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGNLQ
NSYISYWAYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEALH
NHYTQKSLSLSPGK
**Light chain of CD3 full-length antibody CQ54 (SEQ ID NO.36)** 
QTVVTQEPSLTVSPGGTVTLTCGSSTGPVTGGNYPNWVQQKPGQAPRGLIGG
TKFLAPGTPARFSGSLLGGKAALTLSGVQPEDEAEYYCVLWESNRWVFGGGTKLT
VLTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
hIgG1 constant region (SEQ ID NO. 25)
Kappa Chain Constant region (SEQ ID NO. 26) 
LTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC
Heavy chain of OKT3 antibody (SEQ ID NO.27) 
QVQLQQSGAELARPGASVKMSCKASGYTFTRYTMHWVKQRPGQGLEWIGYI
NPSRGYTNYNQKFKDKATLTTDKSSSTAYMQLSSLTSEDSAVYYCARYYDDHYCL
DYWGQGTTLTVSSAKTTAPSVYPLAPVCGGTTGSSVTLGCLVKGYFPEPVTLTWNS
GSLSSGVHTFPAVLQSDLYTLSSSVTVTSSTWPSQSITCNVAHPASSTKVDKKIEPRP
KSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNHYTQK
SLSLSPGK
Light chain of OKT3 antibody (SEQ ID NO.28) QIVLTQSPAIMSASPGEKVTMTCSASSSVSYMNWYQQKSGTSPKRWIYDTSKL ASGVPAHFRGSGSGTSYSLTISGMEAEDAATYYCQQWSSNPFTFGSGTKLEINRADT APTVSIFPPSSEQLTSGGASVVCFLNNFYPKDINVKWKIDGSERQNGVLNSWTDQDS KDSTYSMSSTLTLTKDEYERHNSYTCEATHKTSTSPIVKSFNRNECThe current main applications of CD3 antibodies are bispecific antibodies and activating antibodies. Therefore, the sequences of humanized CD3 antibody heavy chain variable region and light chain variable region (huVH5 and huVL5) were used, and connecting with the scFv of CD19 antibody by a flexible linker (GGGGS) to construct a bispecific antibody targeting CD3 and CD19, named SC30A. Wherein, CD19 was derived from Amgen's marketed bispecific antibody drug blinatumomab, while blinatumomab (named BLMOA) was constructed meanwhile as a control antibody.
**Bispecific antibody SC30A of CD3 and CD19 (SEQ ID NO. 37)** DIQLTQSPASLAVSLGQRATISCKASQSVDYDGDSYLNWYQQIPGQPPKLLIY 
DASNLVSGIPPRFSGSGSGTDFTLNIHPVEKVDAATYHCQQSTEDPWTFGGGTKLEI
KGGGGSGGGGSGGGGSQVQLQQSGAELVRPGSSVKISCKASGYAFSSYWMNWVK
QRPGQGLEWIGQIWPGDGDTNYNGKFKGKATLTADESSSTAYMQLSSLASEDSAV
YFCARRETTTVGRYYYAMDYWGQGTTVTVSSGGGGSEVQLVESGGGLVQPGGSL
KLSCAASGFTFNKYAMNWVRQAPGKGLEWVARIRSKYNNYATYYADSVKDRFTIS
RDDSKNTAYLQMNNLKTEDTAVYYCVRHGNLQNSYISYWAYWGQGTLVTVSSGG
GGSGGGGSGGGGSQTVVTQEPSLTVSPGGTVTLTCGSSTGPVTGGNYPNWVQQKP
GQAPRGLIGGTKFLAPGTPARFSGSLLGGKAALTLSGVQPEDEAEYYCVLWESNRW
VFGGGTKLTVL
**BLMOA (SEQ ID NO.38)**

DIQLTQSPASLAVSLGQRATISCKASQSVDYDGDSYLNWYQQIPGQPPKLLIY
DASNLVSGIPPRFSGSGSGTDFTLNIHPVEKVDAATYHCQQSTEDPWTFGGGTKLEI
KGGGGSGGGGSGGGGSQVQLQQSGAELVRPGSSVKISCKASGYAFSSYWMNWVK
QRPGQGLEWIGQIWPGDGDTNYNGKFKGKATLTADESSSTAYMQLSSLASEDSAV
YFCARRETTTVGRYYYAMDYWGQGTTVTVSSGGGGSDIKLQQSGAELARPGASV
KMSCKTSGYTFTRYTMHWVKQRPGQGLEWIGYINPSRGYTNYNQKFKDKATLTTD
KSSSTAYMQLSSLTSEDSAVYYCARYYDDHYCLDYWGQGTTLTVSSVEGGSGGSG
GSGGSGGVDDIQLTQSPAIMSASPGEKVTMTCRASSSVSYMNWYQQKSGTSPKRWI
YDTSKVASGVPYRFSGSGSGTSYSLTISSMEAEDAATYYCQQWSSNPLTFGAGTKL
ELK

### Example 4 Full length antibody affinity detection

This embodiment involves the binding of full-length antibodies to human monkey protein and their affinity with the human CD3+cell line Jurkat.

### 4.1 Fortebio detection of affinity between full-length CD3 antibodies and human CD3E protein

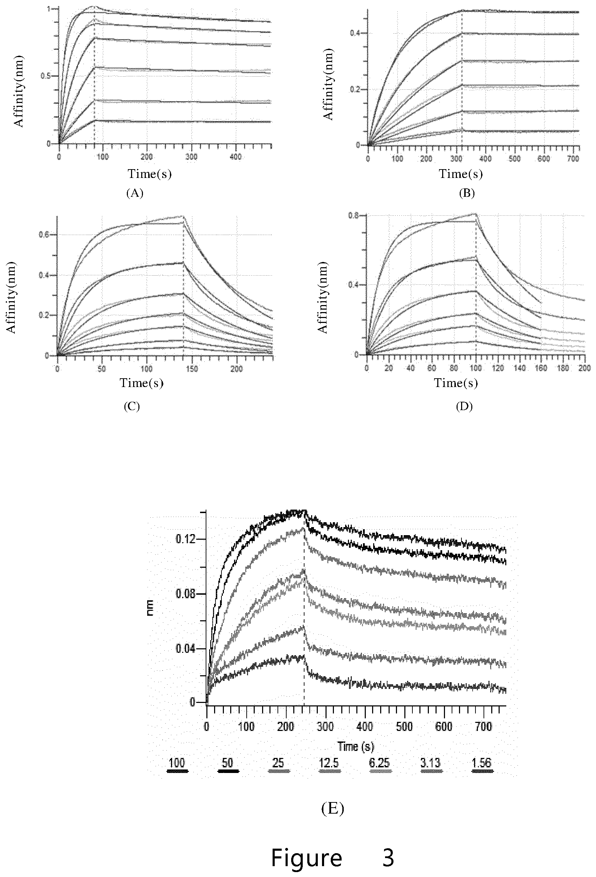

The protein A sensor was selected to immobilize CD3 antibody by using the fortebio method. CD3E was diluted by gradient dilution from 100nM for 7 gradients. The affinity was as shown in Figure 3.

The calculated affinity was shown in Table 3:

**Table 3. Affinity between full-length CD3 antibodies and human CD3E protein**

| Antibody NO. | KD (M) | ka (1/Ms) | Kdis (1/s) |
|---|---|---|---|
| CQ50 | 5.16E-08 | 3.08E+05 | 1.59E-02 |
| CQ51 | 6.24E-08 | 2.06E+05 | 1.29E-02 |
| CQ52 | 3.11E-09 | 1.07E+04 | 3.34E-05 |
| CQ53 | 2.02E-09 | 9.44E+04 | 1.91E-04 |
| CQ54 | 0.97E-9 | 6.21E+05 | 6.04E-04 |

The calculated affinity of CQ50 is 5.16×10⁻⁸M; The affinity of CQ51 is 6.24×10⁻⁸M;

The affinity of CQ52 is 3.11×10⁻⁹M; The affinity of CQ53 is 2.02×10⁻⁹M;

The affinity of CQ53 is 0.97×10⁻⁹M.

### 4.2 Detection of binding between full-length CD3 antibody and monkey CD3E using ELISA

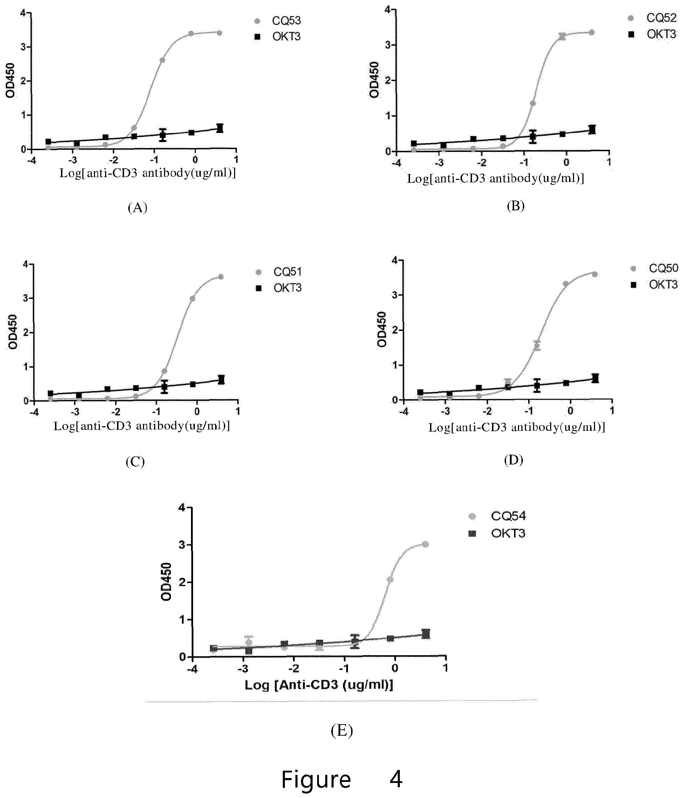

The results are shown in Figure 4,
CQ50 was detected by EC50=0.2004 µg/ml,
CQ51was detected by EC50=0.3385 µg/ml,
CQ52 was detected by EC50=0.195 µg/ml,
CQ53 was detected by EC50=0.0823 µg/ml,
CQ54 was detected by EC50=0.375 µg/ml.

The test results show that CQ50, CQ51, CQ52, CQ53, and CQ54 can bind to monkey CD3E, while OKT3 cannot.

### 4.3 Binding to human CD3 positive cells

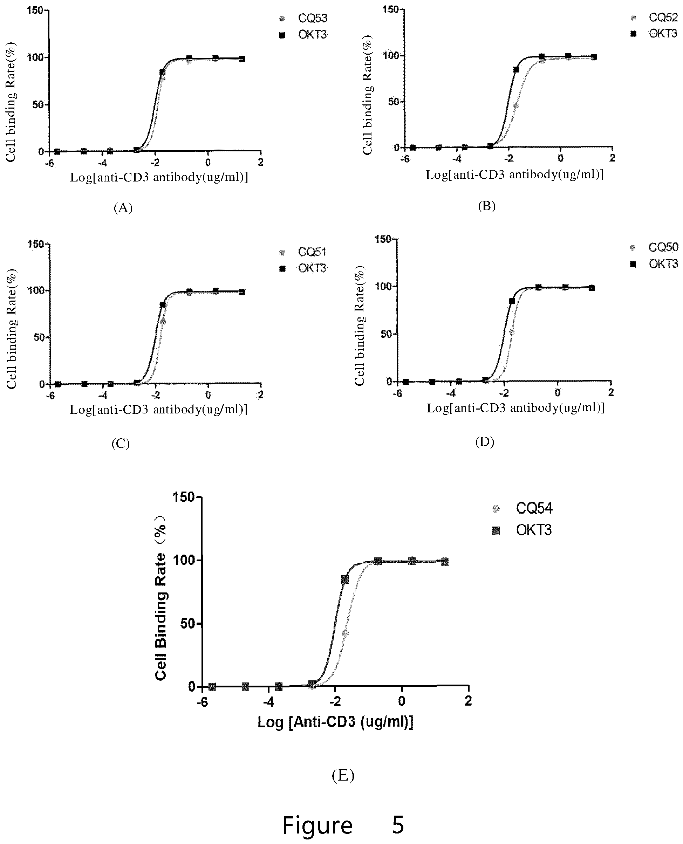

4X10⁵ Jurkat cells was taken to gradient diluted CD3 antibodies, incubated for 1 hour, and the cells were washed 3 times with PBS. Anti hFC-APC (purchased from Jackson immunology) was added, and the sample was performed flow cytometry analysis. The result of drawing the S-curve is shown in Figure 5.

For the EC50 calculated,
The CQ50 obtained is 0.01947 µg/ml, OKT3 is 0.01 µg/ml. The affinity between the two and cells is not significantly different;
The CQ51 obtained is 0.0158 µg/ml, OKT3 is 0.01 µg/ml. The affinity between the two and cells is not significantly different;
The CQ52 obtained is 0.02167 µg/ml, OKT3 is 0.01 µg/ml. The affinity between the two and cells is not significantly different;
The CQ53 obtained is 0.013 µg/ml, OKT3 is 0.01 µg/ml. The affinity between the two and cells is not significantly different;
The CQ54 obtained is 0.023 µg/ml, OKT3 is 0.01 µg/ml. The affinity between the two and cells is not significantly different.

### Example 5 Initial testing of epitope of full-length CD3 antibody

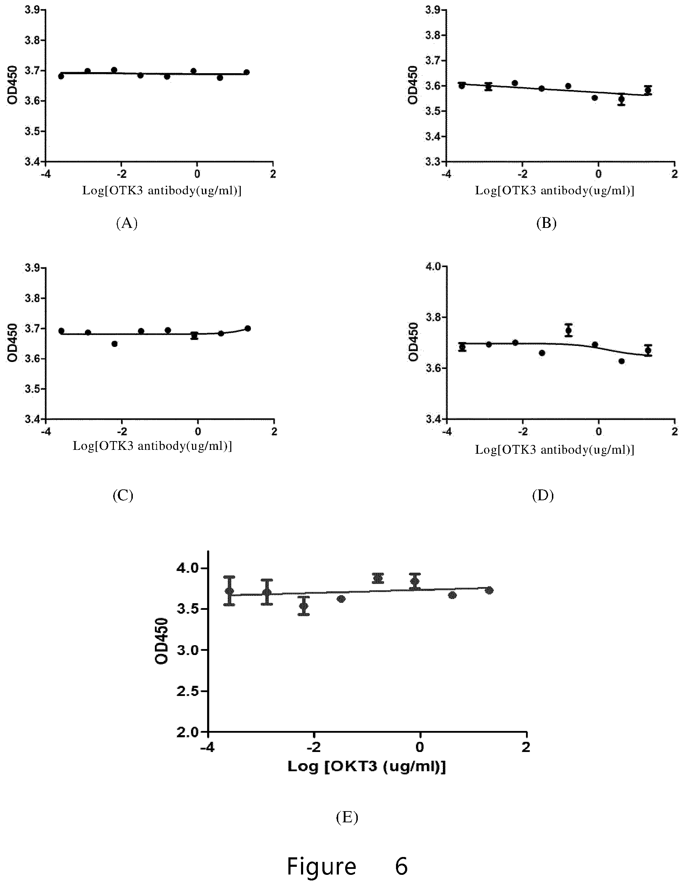

For the functional differences between full-length CD3 antibodies and OKT3 are not significant, this Example mainly investigates whether the binding of full-length CD3 antibodies and OKT3 antibodies to the epitope of CD3E is consistent. Specifically, the plate was coated with the full-length CD3 antibody, and a certain amount of HIS labeled CD3E (C578 Novoprotein) protein was added. Referring to the EC90 value detected by ELISA, gradient diluted OKT3 antibody was added and Anti-HIS secondary antibody (purchased from Biogene) was added for detection. The results are shown in Figure 6.
From the results, there is no competitive relationship between CQ50 and OKT3, and the epitopes of the two antibodies are inconsistent;
There is no competitive relationship between CQ51 and OKT3, and the epitopes of the two antibodies are inconsistent;
There is no competitive relationship between CQ52 and OKT3, and the epitopes of the two antibodies are inconsistent;
There is no competitive relationship between CQ53 and OKT3, and the epitopes of the two antibodies are inconsistent.

There is no competitive relationship between CQ54 and OKT3, and the epitopes of the two antibodies are inconsistent.

### Example 6: Full-length antibodies induce PBMC activation

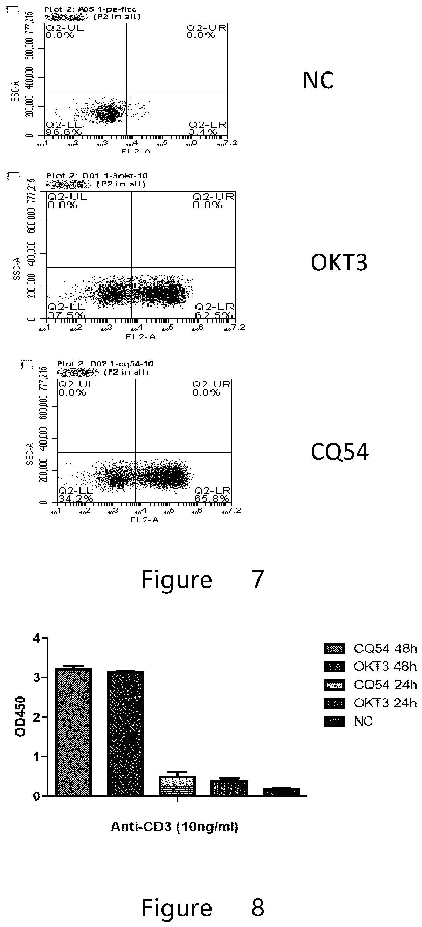

This embodiment relates to the change of expression of CD69 and the IFNγ secretion of early T cell activation markers, when CD3 antibodies were activated in PBMC culture. Specifically, PBMCs from volunteers were isolated using lymphocyte isolation solution, inoculated onto 96 well plates at a density of 1×10⁶/ml, added with 10ng/ml CD3 antibody, cultured overnight, and flow cytometry was used to detect the proportion of CD69+T cells. The results are shown in Figure 7.

The results show that both OKT3 and CQ54 could activate T cells, and in terms of CD69 positivity, the positivity rate of activated CQ54 reached 65.8%, while OKT3 reached 62.5%. The difference of early T cell activation between the two antibodies is not significant.

After PBMC separation, 10ng/ml CD3 antibody was added. After culturing for 24 and 48 hours, samples were taken andIFNγ ELISA Kit was used for detecting IFNγ. The expression is shown in Figure 8.

The results indicate that the effect of CQ54 and OKT3 on the secretion of IFNγ is not different.

### Example 7 Affinity detection of bispecific antibodies

This embodiment involves the binding of bispecific antibodies to two human target proteins and the binding to monkey CD3 protein.

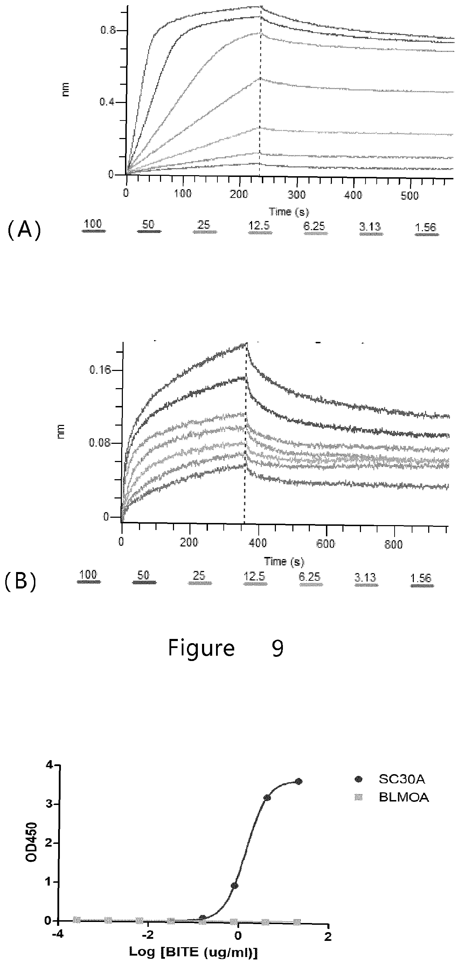

Fortebio's method was used and protein A sensors was selected. FC labeled recombinant human CD3E (CP19 Novoprotein) and human CD19 (C572 Novoprotein) were immobilized separately. The bispecific antibody SC30A was subjected to gradient dilution. The affinity diagrams are shown in Figure 9 (A and B).

The calculated affinity is shown in Table 4:

**Table 4. Affinity test results of bispecific antibodies**

| | KD (M) | ka (1/Ms) | kdis (1/s) |
|---|---|---|---|
| CD3 | 1.48E-09 | 3.20E+05 | 4.74E-04 |
| CD19 | 5.02E-10 | 1.31E+06 | 6.56E-04 |

The results indicate that the bispecific antibody SC30A has affinity for both targets.

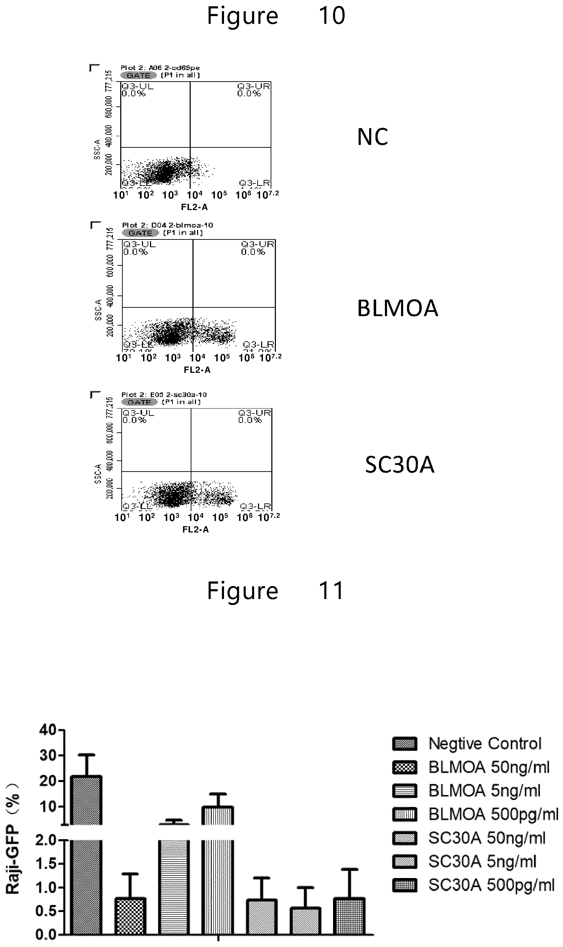

The binding of bispecific antibodies (SC30A and BLMOA) to monkey CD3E was detected by ELISA. The results are shown in Figure 10.

The test results show that SC30A can bind to monkey CD3E, and EC50 was detected that EC50=1.243 µg/ml. And the reference molecule BLMOA cannot bind.

### Example 8: Bispecific antibody induces early activation of PBMC

This embodiment involves changes in the expression of CD69, an early T cell activation marker, when CD3 bispecific antibodies are activated in PBMC culture. Specifically, PBMCs from volunteers were isolated using lymphocyte isolation solution, inoculated onto 96 well plates at a density of 1×10⁶/ml, added with 10ng/ml CD3 antibody, cultured overnight, and flow cytometry was used to detect the proportion of CD69+T cells. The results are shown in Figure 11.

The results show that both SC30A and BLMOA can activate T cells, and the positive rate of CD69 reached 20.7% after SC30A activation, while BLMOA reached 21.9%. The difference in early T cell activation between the two antibodies is not significant.

### Example 9 Bispecific antibody mediated tumor cell killing

This embodiment relates to the killing of CD19+tumor cells Raji by T cells mediated by bispecific antibodies in both activated and inactive T cell states. Specifically, whole blood samples were taken from volunteers, and PBMCs were separated using lymphocyte isolation medium. Cells were cultured with 10ng/ml OKT3 and 100ng/ml IL-2, and cell counts were performed every 3 days until the 10th day. Cell phenotypes were detected to determine that the CD3+cell ratio was greater than 90%. Activated PBMCs and Raj GFP cells were added according to the target effect ratio of 2:1, and gradient diluted bispecific antibodies were added at concentrations of 50ng/ml, 5ng/ml, and 500pg/ml, respectively. The cells were killed overnight, using flow cytometry and fluorescence scanning was used to detect the killing effect.

### 9.1 Flow detection

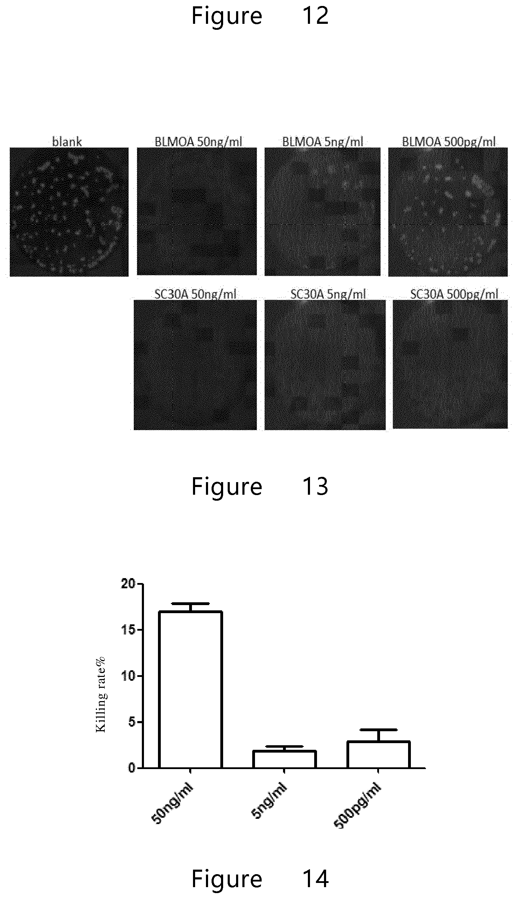

As shown in Figure 12 and Table 5, in the 50ng/ml experiment, there was no significant difference between BLMOA and SC30A in the results of the three volunteer experiments; However, in the 500pg/ml experiment, the proportion of remaining Raji GFP mediated by BLMOA was 6.9%, 19.9%, and 2.8%, respectively, while in the SC30A group, the remaining Raji GFP was 0.3%, 2.0%, and 0.0%, respectively; In the experiment of 5ng/ml, the proportion of remaining Raji GFP mediated by BLMOA was 1.6%, 6.8%, and 0.6%, respectively. In the SC30A group, the remaining Raji GFP was 0.3%, 1.4%, and 0.0%, respectively.

**Table 5. Flow detection results**

| | **Raji -GFP (%)** | | | |
|---|---|---|---|---|
| Experimental group | Sample 1 | Sample 2 | Sample 3 | Average |
| Blank control | 17.8 | 38.1 | 9.9 | 21.9 |
| BLM0A 50ng/m] | 0.4 | 1.8 | 0.1 | 0.8 |
| BLMOA 5ng/ml | 1.6 | 6.8 | 0.6 | 3.0 |
| BLMOA 500pg/ml | 6.9 | 19.9 | 2.8 | 9.9 |
| SC30A 50ng/ml | 0.6 | 1.6 | 0 | 0.7 |
| SC30A 5ng/ml | 0.3 | 1.4 | 0 | 0.6 |
| SC30A 500pg/ml | 0.3 | 2.0 | 0 | 0.8 |

In summary, it indicates that SC30A has more advantages than BLMOA in mediating the activation of PBMC to kill tumor cells.

### 9.2 Fluorescence scanning detection

The residue of Raji GFP was observed using fluorescence scanning of the entire well.

As shown in Figure 13, the bright white spots in the figure are green fluorescence. It can be clearly seen that under the treatment of 500pg/ml BLMOA, there are still many bright white spots of Raji GFP remaining, while under the treatment of 500pg/ml SC30A, almost no bright white spots of Raji GFP can be observed. Therefore, the killing ability mediated by SC30A is superior to BLMOA.

When the separated PBMC is not activated, it was directly mixed with Raji GFP in an efficiency target ratio of 5:1. Gradient dilution was added while adding gradient diluted bispecific antibody SC30A, with concentrations of 50ng/ml, 5ng/ml, and 500pg/ml, respectively. The cells were killed for 48 hours, the killing efficiency diagram is shown in Figure 14.

### Example 10: Effect of B cells on bispecific antibody mediated activation of PBMC

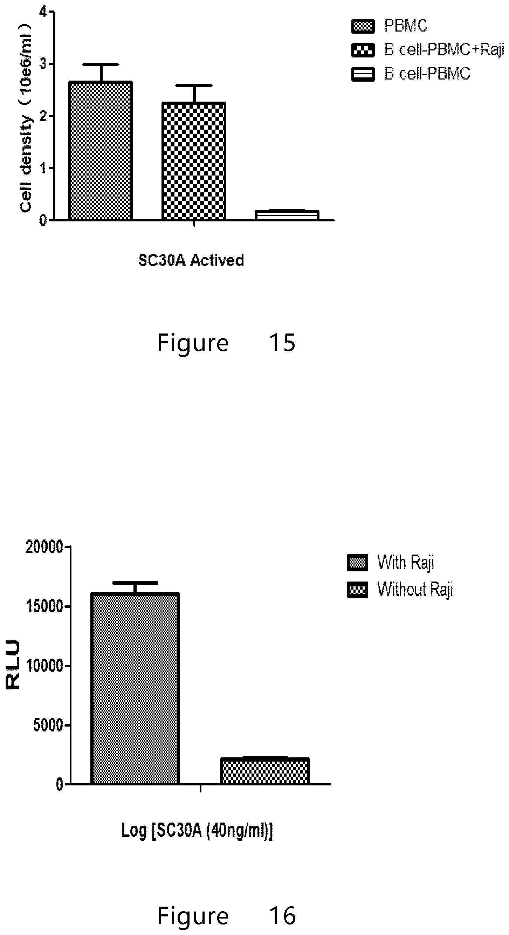

This embodiment involves studying the safety of bispecific antibodies, observing the activation of T cells by bispecific antibodies in the presence and absence of target cells. Specifically, PBMCs were isolated, magnetic beads coated with CD19 antibodies to remove CD19+B cells from the PBMCs to make a B cell-PBMC group. On this basis, 5% Raji cells were added as a supplement to create a B cell-PBMC Raji group. Both the normal and untreated PBMC groups were cultured with SC30A, and after 5 days of culture, cell counts were performed to detect cell proliferation. The results are shown in Figure 15.

The above results indicate that the activation of PBMC by SC30A depends on the target cells.

### Example 11: Intracellular signaling mediated by bispecific antibodies dependent on target cells

This embodiment relates to the downstream signal changes of NFAT transcription factors induced by bispecific antibodies in T cells, and whether this signal also depends on CD19+cells. Specifically, a Jurkat-NFAT-Luc reporter gene cell line (XCC20 Novoprotein) was constructed, in which 40ng/ml of SC30A was added, with one group containing target cell Raji and the other not. After 4 hours of reaction, the cells were lysed to detect luciferase activity. The detection results are shown in Figure 16.

From the experimental results, it can be seen that the signal of adding Raji cells is significantly stronger than that of not adding Raji, and the activation signal of SC30A depends on the target cells.

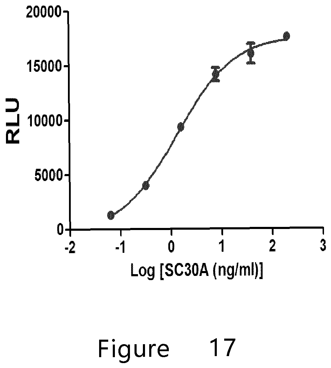

In the presence of target cells, the activity of SC30A was detected. The results are shown in Figure 17.

By using the reporter gene method, the activity of SC30A was detected, and the EC50 was calculated as 1.359ng/ml.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

## Claims

1. An anti-CD3 humanized antibody, wherein the antibody comprises a heavy chain variable region and a light chain variable region, wherein the heavy chain variable region and light chain variable region comprises six complementary determining regions CDRs selected from the following group:
(A5) three complementary determining regions VH-CDRs of the heavy chain variable region and three complementary determining regions VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH5-CDR3: HGNFGNTYISYWAY, | SEQ ID NO.29 |
| HuVL5 CDR1: TGAVTSGNY, | SEQ ID NO.30 |
| HuVL-CDR2: GTK, | SEQ ID NO.5 |
| HuVL5 CDR3: VLWYSKRW, | SEQ ID NO.31; |
(A1) three complementary determining regions VH-CDRs of the heavy chain variable region and three complementary determining regions VL-CDRs of the light chain variable region:
| | |
|---|---|
| HuVH-CDR1: GFTFNKYA, | SEQ ID NO. 1 |
| HuVH-CDR2: IRSKYNNYAT, | SEQ ID NO. 2 |
| HuVH4-CDR3: HGNFGNSYISYWEY, | SEQ ID NO. 3 |
| HuVL-CDR1: TGAVTSGNY, | SEQ ID NO. 4 |
| HuVL-CDR2: GTK, | SEQ ID NO. 5 |
| HuVL4-CDR3: VLWNSNRW, | SEQ ID NO. 6; |
(A2) three complementary determining regions VH-CDRs of the heavy chain variable region and three complementary determining regions VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH3-CDR3: HGNFGNSYISYWRY, | SEQ ID NO.10 |
| HuVL-CDR1: TGAVTSGNY, | SEQ ID NO.4 |
| HuVL-CDR2: GTK, | SEQ ID NO.5 |
| HuVL3-CDR3: VLWYSGRW, | SEQ ID NO.11; |
(A3) three complementary determining regions VH-CDRs of the heavy chain variable region and three complementary determining regions VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH2-CDR3: HGNFGNSYISYWQY, | SEQ ID NO.15 |
| HuVL-CDR1: TGAVTSGNY, | SEQ ID NO.4 |
| HuVL-CDR2: GTK, | SEQ ID NO.5 |
| HuVL2-CDR3: VLWRSNRW, | SEQ ID NO.16; |
or
(A4) three complementary determining regions VH-CDRs of the heavy chain variable region and three complementary determining regions VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH1-CDR3: HGNFGNTYISYWAY, | SEQ ID NO.20 |
| HuVL-CDR1: TGAVTSGNY, | SEQ ID NO.4 |
| HuVL-CDR2: GTK, | SEQ ID NO.5 |
| HuVL1-CDR3: VLWYSKRW, | SEQ ID NO.21; |
wherein, any one of the above amino acid sequences also includes a derivative sequence that is optionally with at least one amino acid added, deleted, modified, and/or substituted, and is capable of retaining the binding affinity of CD3.

2. The antibody of claim 1, wherein the antibody comprises a heavy chain and a light chain, wherein the heavy chain of the antibody comprises three complementary determining regions VH-CDR and a heavy chain framework region for connecting VH-CDR, and the light chain of the antibody comprises three complementary determining regions VL-CDRs and a light chain framework region for connecting VL-CDRs.

3. The antibody of claim 1, wherein the antibody is a single chain antibody.

4. A bispecific antibody, wherein the bispecific antibody comprises:
the first antigen binding domain D1; and
the second antigen binding domain D2;
wherein, D1 specifically binds to the target molecule CD3 protein; D2 specific binding target molecule CD19 protein;
wherein, D1 is an antibody or antigen-binding fragment that specifically binds to CD3 protein;
D2 is an antibody or antigen-binding fragment that specifically binds to CD19 protein;
wherein, D1 comprises a heavy chain variable region and a light chain variable region, wherein the heavy chain variable region and the light chain variable region comprise six complementary determining regions CDRs selected from the following group:
(A5) the three complementary determining regions VH-CDRs of the heavy chain variable region and VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH5-CDR3: HGNFGNTYISYWAY, | SEQ ID NO.29 |
| HuVL5 CDR1: TGAVTSGNY, | SEQ ID NO.30 |
| HuVL-CDR2: GTK, | SEQ ID NO.5 |
| HuVL5 CDR3: VLWYSKRW, | SEQ ID NO.31; |
(A1) the three complementary determining regions VH-CDRs of the heavy chain variable region and VL-CDRs of the light chain variable region:
| | |
|---|---|
| HuVH-CDR1: GFTFNKYA, | SEQ ID NO. 1 |
| HuVH-CDR2: IRSKYNNYAT, | SEQ ID NO. 2 |
| HuVH4-CDR3: HGNFGNSYISYWEY, | SEQ ID NO. 3 |
| HuVL-CDR1: TGAVTSGNY, | SEQ ID NO. 4 |
| HuVL-CDR2: GTK, | SEQ ID NO. 5 |
| HuVL4-CDR3: VLWNSNRW, | SEQ ID NO. 6; |
(A2) the three complementary determining regions VH-CDRs of the heavy chain variable region and VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH3-CDR3: HGNFGNSYISYWRY, | SEQ ID NO.10 |
| HuVL-CDR1: TGAVTSGNY, | SEQ ID NO.4 |
| HuVL-CDR2: GTK, | SEQ ID NO.5 |
| HuVL3-CDR3: VLWYSGRW, | SEQ ID NO.11; |
(A3) the three complementary determining regions VH-CDRs of the heavy chain variable region and VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH2-CDR3: HGNFGNSYISYWQY, | SEQ ID NO.15 |
| HuVL-CDR1: TGAVTSGNY, | SEQ ID NO.4 |
| HuVL-CDR2: GTK, | SEQ ID NO.5 |
| HuVL2-CDR3: VLWRSNRW, | SEQ ID NO.16; |
or
(A4) the three complementary determining regions VH-CDRs of the heavy chain variable region and VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH1-CDR3: HGNFGNTYISYWAY, | SEQ ID NO.20 |
| HuVL-CDR1: TGAVTSGNY, | SEQ ID NO.4 |
| HuVL-CDR2: GTK, | SEQ ID NO.5 |
| HuVL1-CDR3: VLWYSKRW, | SEQ ID NO.21; |
wherein, the structure of the antigen binding fragment is selected from the following group: (i) Fab fragment; (ii) F (ab ') ₂ fragment; (iii) Fd fragments; (iv) Fv fragments; (v) ScFv molecules; or (vi) dAb fragments.

5. A recombinant protein, wherein the recombinant protein comprises:
(i) the antibody of claim 1 or the bispecific antibody of claim 4; and
(ii) optional tag sequence that facilitate expression and/or purification.

6. A CAR construct, wherein the scFv segment of the antigen-binding region of the CAR construct is a binding region that specifically binds to CD3 and the scFv segment has a heavy chain variable region and a light chain variable region, wherein the heavy chain variable region and a light chain variable region comprises six complementary determining regions CDRs selected from the following group:
(A5) the three complementary determining regions VH-CDRs of the heavy chain variable region and VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH5-CDR3: HGNFGNTYISYWAY, | SEQ ID NO.29 |
| HuVL5 CDR1: TGAVTSGNY, | SEQ ID NO.30 |
| HuVL-CDR2: GTK, | SEQ ID NO.5 |
| HuVL5 CDR3: VLWYSKRW, | SEQ ID NO.31; |
(A1) the three complementary determining regions VH-CDRs of the heavy chain variable region and VL-CDRs of the light chain variable region:
| | |
|---|---|
| HuVH-CDR1: GFTFNKYA, | SEQ ID NO. 1 |
| HuVH-CDR2: IRSKYNNYAT, | SEQ ID NO. 2 |
| HuVH4-CDR3: HGNFGNSYISYWEY, | SEQ ID NO. 3 |
| HuVL-CDR1: TGAVTSGNY, | SEQ ID NO. 4 |
| HuVL-CDR2: GTK, | SEQ ID NO. 5 |
| HuVL4-CDR3: VLWNSNRW, | SEQ ID NO. 6; |
(A2) the three complementary determining regions VH-CDRs of the heavy chain variable region and VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH3-CDR3: HGNFGNSYISYWRY, | SEQ ID NO.10 |
| HuVL-CDR1: TGAVTSGNY, | SEQ ID NO.4 |
| HuVL-CDR2: GTK, | SEQ ID NO.5 |
| HuVL3-CDR3: VLWYSGRW, | SEQ ID NO.11; |
(A3) the three complementary determining regions VH-CDRs of the heavy chain variable region and VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH2-CDR3: HGNFGNSYISYWQY, | SEQ ID NO.15 |
| HuVL-CDR1: TGAVTSGNY, | SEQ ID NO.4 |
| HuVL-CDR2: GTK, | SEQ ID NO.5 |
| HuVL2-CDR3: VLWRSNRW, | SEQ ID NO.16; |
or
(A4) the three complementary determining regions VH-CDRs of the heavy chain variable region and VL-CDRs of the light chain variable region:
| | |
|---|---|
| HuVH-CDR1: GFTFNKYA, | SEQ ID NO.1 |
| HuVH-CDR2: IRSKYNNYAT, | SEQ ID NO.2 |
| HuVH1-CDR3: HGNFGNTYISYWAY, | SEQ ID NO.20 |
| HuVL-CDR1: TGAVTSGNY, | SEQ ID NO.4 |
| HuVL-CDR2: GTK, | SEQ ID NO.5 |
| HuVL1-CDR3: VLWYSKRW, | SEQ ID NO.21. |

7. A recombinant immune cell, wherein the immune cell expresses the exogenous CAR construct of claim 6.

8. An antibody-drug conjugate, wherein the antibody-drug conjugate comprises:
(a) the antibody portion is selected from the following group: the antibody of claim 1, the bispecific antibody of claim 4, or the recombinant protein of claim 5, or a combination thereof; and
(b) a coupling moiety coupled to the antibody moiety, and the coupling moiety is selected from the group consisting of a detectable label, a drug, a toxin, a cytokine, a radionuclide, an enzyme, or a combination thereof.

9. Use of an active ingredient, wherein the active ingredient is selected from the following group: the antibody of claim 1, the bispecific antibody of claim 4, or the recombinant protein of claim 5, the CAR construct of claim 6, the immune cell of claim 7, the antibody drug conjugate of claim 8, or combination thereof, wherein the active ingredient is used for:
(a) preparing testing reagents or test kits;
(b) preparing drugs or preparations for the prevention and/or treatment of CD3 related diseases; and/or
(c) preparing drugs or preparations for the prevention and/or treatment of CD3 related cancers or tumors.

10. A pharmaceutical composition, wherein the pharmaceutical composition comprises:
(i) an active ingredient, wherein the active ingredient is selected from the group consisting of: the antibody of claim 1, the bispecific antibody of claim 4, or the recombinant protein of claim 5, the CAR construct of claim 6, the immune cells of claim 7, the antibody drug conjugate of claim 8, or combination thereof; and
(ii) a pharmaceutically acceptable carrier.

11. A polynucleotide, wherein the polynucleotide encodes a polypeptide selected from group consisting of:
(1) the antibody of claim 1; or
(2) the bispecific antibody of claim 4;
(3) the recombinant protein of claim 5;
(4) the CAR construct of claim 6.

12. A the vector comprising the polynucleotide of claim 11.

13. A genetically engineered host cell, wherein the host cell contains the vector of claim 12 or the genome thereof is integrated with the polynucleotide of any one of claims 11.

14. A method for in *vitro* non-diagnostic detection of CD3 protein in samples , wherein the method comprises the steps:
*(1) in vitro* contacting the sample with the antibody of any one of claims 1;
(2) detecting the formation of an antigen-antibody complex, wherein the formation of a complex indicates the presence of CD3 protein in the sample.
